# EUROPEAN PATENT SPECIFICATION

(11) **EP 1 211 243 B1**
(45) Date of publication and mention of the grant of the patent: **14.10.2009**
(21) Application number: 02005248.6
(22) Date of filing: 06.06.1997
(51) Int. Cl.: C07D 211/32, A61K 31/445, A61P 25/28

(54) **Polymorphs of donepezil hydrochloride and process for production**
Polymorphe von Donepezil-Hydrochlorid und Verfahren zur Herstellung
Polymorphes de l'hydrochlorure de donépézile et procédé de préparation

(30) Priority: 07.06.1996 JP 14629396; 27.12.1996 WO PCT/JP96/03881; 30.12.1996 US 774802
(43) Date of publication of application: 05.06.2002
(62) Divisional of application: 97924337.5
(73) Proprietor: Eisai R&D Management Co., Ltd., Tokyo 112-8088 (JP)
(72) Inventor: Watanabe, Hideaki, Ushiku-shi, Ibaraki 300-12 (JP); Imai, Akio, Kashima-gun, Ibaraki 314-0132 (JP); Kajima, Takashi, Ryugasaki-shi, Ibaraki 301 (JP); Ishihama, Yasushi, Kitasoma-gun, Ibaraki 302-0124 (JP); Ohtsuka, Akiyo, Tsukuba-shi, Ibaraki 305 (JP); Tanaka, Tomohide, Kashima-gun, Ibaraki 314-02 (JP); Nabaru, Yukio, Kashima-gun, Ibaraki 314-04 (JP)
(74) Representative: HOFFMANN EITLE

(56) References cited:
- EP-A- 0 296 560
- EP-A- 0 673 927

## Description

### [Field of the invention]

The present invention relates to the stable polymorphs of Donepezil hydrochloride, that is, 1-benzyl-4-[(5,6-dimethoxy-1-indanon)-2-yl]methyl-piperidine hydrochloride, disclosed in the example 4 of US-A 4,895,841 or EP-A 296560, having an excellent efficacy as pharmaceuticals, and industrial processes for producing them.

### [Background of the invention]

Donepezil hydrochloride shows the acetylcholine esterase-inhibitory action and is useful for the treatment of all kinds of senile dementia, in particular being useful for prevention, treatment and amelioration of Alzheimer Disease. Donepezil hydrochloride is administered orally as usual and it may be placed for distribution and storage in a period of time before the administration. It may then be stored at patient's home for about one month at the maximum because of the property of the target disease. The stability of this medicinal substance (bulk pharmaceutical chemicals) against heat and humidity during the storage period is very important. A more stable medicinal substance of Donepezil hydrochloride is, therefore, desired. It is not known, however, that polymorphs of Donepezil hydrochloride exist. No sufficiently stable medicinal substance of Donepezil hydrochloride has been found.

### [Prior Arts]

US-A 4, 895, 841 (corresponding to EP 0 296 560 A) discloses in Example 4 that recrystalization of the crude product mixture of Donepezil hydrochloride from ethanol/isopropyl ether afforded a purified Donepezil hydrochloride. If there is a more stable crystalline form of Donepezil hydrochloride for a long period, it is more practical for distribution and storage.

EP 0 673 927 A, which is a divisional patent application of EP 0 296 560 A, discloses related cyclic amine compounds for use in the treatment of senile dementia.

### [Summary of the invention]

Regarding the foregoing problems, the present inventors have proceeded with extensive research. As a result, it has been found that novel polymorph of Donepezil hydrochloride (IV) can be produced and have an excellent stability, establishing the present invention. The present invention offers the novel polymorph of Donepezil hydrochloride and industrially excellent processes for producing it.

In detail, the present invention relates to the polymorph (IV) of Donepezil hydrochloride represented by the following chemical structure, the polymorphs being specified by the peaks appearing in the powder X-ray diffraction pattern and infrared absorption spectra in potassium bromide. 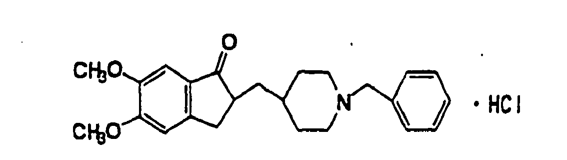 (Method and condition of the measurement of X-ray diffraction patterns)

### (1) Method of the measurement

X-ray diffraction patterns were measured on each 100mg of the samples by the following condition.

### (2) Condition of the measurement

| | | |
|---|---|---|
| Target | ; | Cu |
| Filter | ; | monochro |
| Voltage | ; | 40 KV |
| Current | ; | 20 mA |
| Slit | ; | DS 1, RS 0.15, SS 1 |
| Scan speed | ; | 2 deg/min. |
| Range | ; | 5-30 |
| Step/Sample | ; | 0.02 deg |

### (Method and condition of the measurement of infrared absorption)

Infrared absorption spectra in potassium bromide were measured according to the general method recorded in the Japanese Pharmacopoeia.

### Polymorph (IV)

Peaks in the powder X-ray diffraction pattern are:

| Diffraction angles | Intensity |
|---|---|
| (2θ,°) | (I/I₀) |
| | |
| 6.24 | 15 |
| 9.66 | 12 |
| 11.04 | 22 |
| 12.12 | 24 |
| 12.54 | 67 |
| 12.76 | 61 |
| 13.98 | 27 |
| 14.42 | 15 |
| 14.88 | 11 |
| 16.34 | 12 |
| 17.46 | 100 |
| 18.12 | 25 |
| 18.60 | 32 |
| 19.06 | 15 |
| 19.98 | 74 |
| 20.42 | 41 |
| 20.62 | 34 |
| 21.30 | 48 |
| 21.80 | 63 |
| 22.58 | 78 |
| 23.04 | 46 |
| 24.00 | 32 |
| 24.54 | 49 |
| 25.14 | 90 |
| 25.36 | 99 |
| 26.06 | 34 |
| 28.10 | 41 |
| 28.58 | 39 |
| 29.30 | 31 |
| 29.44 | 28. |

Wave numbers (cm⁻¹) of infrared absorption spectra in potassium bromide are:
401, 431, 459, 467, 490, 506, 518, 561, 586, 606, 631, 651, 709, 758, 766, 857, 944, 1009, 1041, 1106, 1119, 1132, 1213, 1225, 1265, 1304, 1318, 1429, 1458, 1470, 1500, 1589, 1605, 1630, 1647, 1683, 2562, 2577, 2608, 2634, 2689, 2717, 2836, 2924, 2949, 2989, 3007, 3032, 3061, 3322, 3376, 3422 cm⁻¹.

The melting point of the novel Polymorph (IV) disclosed in the present invention is different from that of example 4 in US-A-4,895,841.

A melting point in US-P-4,895,841 is 211-212°C (decomposition).

| | |
|---|---|
| Melting point of the polymorph (I): | 225-226°C (decomposition), |
| Melting point of the polymorph (II): | 224-226°C (decomposition), |
| Melting point of the polymorph (IV): | 226-228°C (decomposition), |
| [Melting point of the amorphous form: | 220-222° C (decomposition), |

Furthermore, the thermogravimetric and differential thermal analysis (TG-DTA) of the present polymorphs measured under the following condition show different patterns from the prior art. It is noted accordingly that their crystalline forms are completely different from the prior art.

### {Method and condition of the thermogravimetric and differential thermal analysis (TG-DTA)}

About 3 to 6 mg of Samples were taken and subjected to thermal analysis under the following condition.

| | | |
|---|---|---|
| Reference | ; | empty |
| Scan speed | ; | 5°C/min. |
| Sampling | ; | 0.7 sec. |
| Upper limit | ; | 300° C |
| Lower limit | ; | Room temperature. |

Detailed processes for preparing the novel polymorph are as follows. In these processes, "Donepezil" means a free base of the Donepezil hydrochloride, i.e., 1-benzyl-4-[(5,6-dimethoxy-1-indanon)-2-yl]methylpiperidine.
(4) A process for preparing the polymorph (IV) is:
   (4-1) Humidification of the polymorph (II), which is characterized below:
      Process (4-1) is illustrated in Example 16.

### (2) Polymorphs (II)

Peaks in the powder X-ray diffraction pattern are:

| Diffraction angles | Intensity |
|---|---|
| (2θ,°) | (I/I₀) |
| 7.40 | 8 |
| 9.88 | 100 |
| 12.36 | 13 |
| 15.54 | 40 |
| 16.10 | 38 |
| 16.22 | 38 |
| 16.48 | 35 |
| 17.30 | 17 |
| 18.04 | 20 |
| 18.44 | 17 |
| 18.84 | 19 |
| 19.34 | 19 |
| 19.84 | 47 |
| 21.16 | 24 |
| 22.40 | 19 |
| 23.18 | 33 |
| 24.02 | 22 |
| 24.92 | 25 |
| 25.72 | 27 |
| 26.-40 | 18 |
| 27.22 | 14. |

Wave numbers (cm⁻¹) of infrared absorption spectra in potassium bromide are:
699, 748, 762, 845, 947, 1009, 1035, 1067, 1103, 1118, 1129, 1174, 1193, 1206, 1222, 1247, 1267, 1317, 1365, 1422, 1436, 1456, 1465, 1502, 1592, 1607, 1688, 2412, 2489, 2627, 2846, 2868, 2913, 2928, 3435 cm⁻¹.

Further Detailed processes for preparing the novel polymorphs are as follows.
(4) A process for preparing the polymorph (IV) are:
   (4-2) Dissolving Donepezil in hydrochloric acid, followed by filtration of the separated crystals,
   (4-3) Dissolving Donepezil in hydrochloric acid, followed by addition of tetrahydrofuran,
   (4-4) Dissolving Donepezil in a mixture of water and
   tetrahydrofuran, followed by addition of hydrochloric acid or hydrogen chloride,
   (4-5) Dissolving Donepezil in methanol, toluene or n-hexane, followed by addition of hydrochloric acid,
   (4-6) Dissolving Donepezil in a mixture of methanol and
   hydrochloric acid,
   (4-7) Dissolving Donepezil in water, followed by addition of hydrochloric acid or hydrogen chloride,
   (4-8) Recrystalization of Donepezil hydrochloride from water,
   (4-9) Humidification of the polymorph (II) of Donepezil hydrochloride; or
   (4-10) Humidification of the amorphous form of Donepezil hydrochloride.

Process (4-4) is preferred. This process is illustrated in Example 48.

Process (4-8) is also preferred. This process is illustrated in Example 53.

Polymorph (II), which is not in accordance with the appended claims, but which can be used for obtaining polymorph (IV), may itself be prepared as follows:
(2) Processes for preparing the polymorph (II) are:
   (2-1) Dissolving Donepezil hydrochloride in ethanol, followed by addition of diethyl ether or diisopropyl ether,
   (2-2) Dissolving Donepezil hydrochloride in ethanol, followed by addition of diisopropyl ether, then filtration of the crystals after 10 to 30 minutes from the separation,
   (2-3) Dissolving Donepezil and hydrochloric acid or hydrogen chloride in ethanol, followed by addition of diethyl ether,
   (2-4) Dissolving Donepezil in ethanol, followed by addition of hydrochloric acid or hydrogen chloride, then concentration,
   (2-5) Dissolving Donepezil in ethanol, followed by addition of hydrochloric acid or hydrogen chloride, and diisopropyl ether successively; or
   (2-6) Dissolving Donepezil in ethanol, followed by addition of hydrochloric acid or hydrogen chloride, and diisopropyl ether successively, then filtration of the crystals after 10 to 60 minutes, preferably 10 to 30 minutes from the separation.

Process (2-6) is preferred. This process is illustrated in Example 14.
(2-7) Dissolving Donepezil in ethanol, followed by addition of hydrochloric acid or hydrogen chloride and addition of tert-butyl methyl ether successively,
(2-8) Dissolving Donepezil in isopropyl alcohol, acetone or tetrahydrofran, followed by addition of hydrochloric acid or hydrogen chloride,
(2-9) Dissolving Donepezil in methylene chloride, followed by addtion of hydrochloric acid or hydrogen chloride and addition of diisopropyl ether successively,
(2-10) Dissolving Donepezil hydrochloride in ethanol, followed by addition of tert-butyl methyl ether or diisopropyl ether and stirring blow 10°C,
(2-11) Dissolving Donepezil hydrochloride in methylene chloride, followed by addition of tert-butyl methyl ether or diisopropyl ether; or
(2-12) Heating the polymorph (I) or amorphous form of Donepezil hydrochloride.

Process (2-7) is preferred. This process is illustrated in Example 33.

Process (2-10) is also preferred. This process is illustrated in Example 40, 41 and 42 Polymorph (I), which is also not in accordance with the appended claims, but which may be used as a starting material in process (2-12) above, is characterized by the data shown below and it may itself be prepared as follows: (1) Polymorphs (I)

Peaks in the powder X-ray diffraction pattern are:

| Diffraction angles | Intensity |
|---|---|
| (2θ.°) | (I/I₀) |
| 9.94 | 24 |
| 10.60 | 19 |
| 12.66 | 69 |
| 13.12 | 55 |
| 13.66 | 44 |
| 13.86 | 40 |
| 14.92 | 49 |
| 15.26 | 17 |
| 16.08 | 35 |
| 16.86 | 34 |
| 17.50 | 34 |
| 17.58 | 42 |
| 18.42 | 20 |
| 19.28 | 27 |
| 19.80 | 45 |
| 19.94 | 45 |
| 21.22 | 100 |
| 22.00 | 32 |
| 22.54 | 31 |
| 22.98 | 49 |
| 23.60 | 56 |
| 23.78 | 75 |
| 23.92 | 78 |
| 26.46 | 33 |
| 28.02 | 25 |
| 29.50 | 37. |

Wave numbers (cm⁻¹) of infrared absorption spectra in potassium bromide are:
463, 502, 563, 589, 604, 701, 750, 759, 799, 860, 922, 947, 972, 1012, 1038, 1104, 1120, 1128, 1175, 1192, 1218, 1250, 1267, 1316, 1368, 1410, 1433, 1440, 1455, 1472, 1502, 1591, 1606, 1644, 1684; 2412, 2530, 2559, 2595, 2620, 2717, 2840, 2858, 2924, 3004, 3074, 3259, 3373, 3547, 3589 cm⁻¹.
(1) Processes for preparing the polymorph (I) are:
   (1-1) Recrystalization of Donepezil hydrochloride from methanol,
   (1-2) Dissolving Donepezil hydrochloride in methanol, followed by addition of diethyl ether or diisopropyl ether,
   (1-3) Dissolving Donepezil in methanol, followed by addition of hydrochloric acid or hydrogen chloride,
   (1-4) Dissolving Donepezil in ethanol, followed by addition of diisopropyl ether, and hydrochloric acid or hydrogen chloride successively; or
   (1-5) Dissolving Donepezil in ethanol, followed by addition of hydrochloric acid or hydrogen chloride, and diisopropyl ether successively, then filtration of the crystals immediately after separation.

Process (1-5) is preferred. This process is illustrated in Example 7.
(1-6) Dissolving Donepezil in methanol, followed by addition of hydrochloric acid or hydrogen chloride,
(1-7) Dissolving Donepezil in methanol, followed by addition of hydrochloric acid or hydrogen chloride and addition of tert-butyl methyl ether, diisopropyl ether or ethyl acetate successively,
(1-8) Dissolving Donepezil in ethanol, tetrahydrofuran or acetonitrile, followed by addition of hydrochloric acid or hydrogen chloride,
(1-9) Dissolving Donepezil hydrochloride in methanol, followed by addition of tert-butyl methyl ether, ethyl acetate or n-hexane,
(1-10) Recrystalization of Donepezil hydrochloride from ethanol; or
(1-11) Dissolving Donepezil hydrochloride in ethanol, followed by addition of tert-butyl methyl ether.

Process (1-7) is preferred. This process is illustrated in Example 18, 19 and 20.
Process (1-9) is also preferred. This process is illustrated in Example 27, 28 and 29.

The invention further provides a therapeutical composition which comprises a pharmacologically effective amount of Donepezil hydrochloride in the form of polymorph (IV) as above and a pharmacologically acceptable carrier.

The compound in the form of polymorph (IV) of the present invention is effective for treatment, prevention, remission, improvement, etc. of various kinds of senile dementia, particularly senile dementia of the Alzheimer type; cerebrovascular diseases accompanying cerebral apoplexy, e.g. cerebral hemorrhage or cerebral infarcts, cerebral arteriosclerosis, head injury, etc.; and aprosexia, disturbance of speech, hypobulia, emotional changes, attention deficit/hyperactivity disorder, recent memory disturbance, hallucinatory-paranoid syndrome, behavioral changes, etc. accompanying encephalitis, cerebral palsy, etc.

The invention provides a polymorph of Donepezil hydrochloride that can be used in a method for treating a disease accompanied by acetylcholinesterase activity by administering to a human patient a pharmacologically' effective amount of the Donepezil hydrochloride in the form of polymorph (IV) as above for inhibiting the acetylcholinesterase activity.

The invention further provides a therapeutical composition which comprises a pharmacologically effective amount of Donepezil hydrochloride in the form of polymorph (IV) as above and a pharmacologically acceptable carrier.

The compound in the form of polymorph (IV) of the present invention is effective for treatment, prevention, remission, improvement, etc. of various kinds of senile dementia, particularly senile dementia of the Alzheimer type; cerebrovascular diseases accompanying cerebral apoplexy, e.g. cerebral hemorrhage or cerebral infarcts, cerebral arteriosclerosis, head injury, etc.; and aprosexia, disturbance of speech, hypobulia, emotional changes, attention deficit/hyperactivity disorder, recent memory disturbance, hallucinatory-paranoid syndrome, behavioral changes, etc. accompanying encephalitis, cerebral palsy, etc.

Further; the compound in the form of polymorph of the present invention has a strong and highly selective acetylcholinesterase action, which renders the compound of the present invention useful also as a pharmaceutical based on this kind of action.

Specifically, the compound in the form of polymorph of the present invention is effective for, for example, Huntington's chorea, Pick's disease and delayed ataxia or tardive dyskinesia other than senile dementia of the Alzheimer type.

When the compound in the form of polymorph of the present invention is used as a pharmaceutical for these diseases, it may be orally or parenterally administered. In general, it is parenterally administered in the form of injections, such as intravenous, subcutaneous, and intramuscular injections, suppositories, or sublingual tablets. The dose will remarkably vary depending upon the symptom; age, sex, weight, and sensitivity of patients; method of administration; time and intervals of administration and properties, dispensing, and kind of pharmaceutical preparations; kind of effective ingredients, etc., so that there is no particular limitation with respect to the dose. Normally the compound may be administered in a dose of about 1.0 to 300 mg, preferably 1 to 100 mg, per day per adult, ordinarily in one to four portions.

Pharmaceutical preparations in the dosage form of, e.g., injections, suppositories, sublingual tablets, tablets, and capsules are prepared according to a method which is commonly accepted in the art.

In preparing injections, the effective ingredient is blended, if necessary, with a pH modifier, a buffer, a suspending agent, a solubilizing agent, a stabilizer, a tonicity agent, a preservative, etc., followed by preparation of an intravenous, subcutaneous, or intramuscular injection according to an ordinary method. In this case, if necessary, it is possible to lyophilize these preparations according to an ordinary method.

Examples of the suspending agents include methylcellulose, Polysorbate 80, hydroxyethylcellulose, acacia, powdered tragacanth, sodium carboxymethylcellulose, and polyoxyethylene sorbitan monolaurate.

Examples of the solubility agent include polyoxyethylene hydrogenated castor oil, Polysorbate 80, nicotinamide, polyoxyethylene sorbitan monolaurate, Macrogol, and an ethyl ester of castor oil fatty acid.

Examples of the stabilizer include sodium sulfite, sodium metasulfite and ether, and examples of the preservative include methyl p-hydroxybenzoate, ethyl p-hydroxybenzoate, sorbic acid, phenol, cresol, and chlorocresol.

### [Explanation of the drawings]

- Fig. 1: is powder X-ray diffraction pattern of the polymorph (I).
- Fig. 2: is powder X-ray diffraction pattern of the polymorph (II).
- Fig. 3: is powder X-ray diffraction pattern of the polymorph (IV).
- Fig. 4: is powder X-ray diffraction pattern of the Amorphous form.
- Fig. 5: is infrared absorption in potassium bromide of the polymorph (I).
- Fig. 6: is infrared absorption in potassium bromide of the polymorph (II).
- Fig. 7: is infrared absorption in potassium bromide of the polymorph (IV).
- Fig. 8: is infrared absorption in potassium bromide of the amorphous form.
- Fig. 9: is thermogravimetric and differential thermal analysis (TG-DTA) of the polymorph (I).
- Fig. 10: is thermogravimetric and differential thermal analysis (TG-DTA) of the polymorph (II).
- Fig. 11: is thermogravimetric and differential thermal analysis (TG-DTA) of the polymorph (IV).
- Fig. 12: is thermogravimetric and differential thermal analysis (TG-DTA) of the amorphous form.
- Fig. 13: is change of impurity contents for each polymorphs and amorphous form stored at -20°C.
- Fig. 14: is change of impurity contents for each polymorphs and amorphous form stored at 40°C.
- Fig. 15: is change of impurity contents for each polymorphs and amorphous form stored at 60°C.
- Fig. 16: is change of impurity contents for each polymorphs and amorphous form stored at 80°C.
- Fig. 17: is water contents for each polymorphs and amorphous form stored at 25°C under various relative humidity condition.

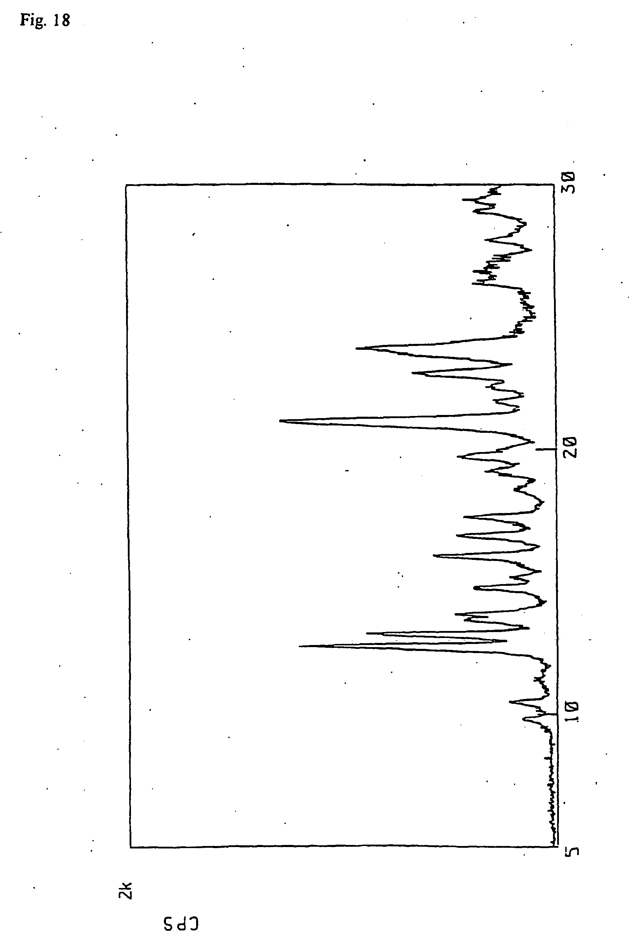

- Fig. 18: is clear powder X-ray diffraction pattern of the polymorph (I).

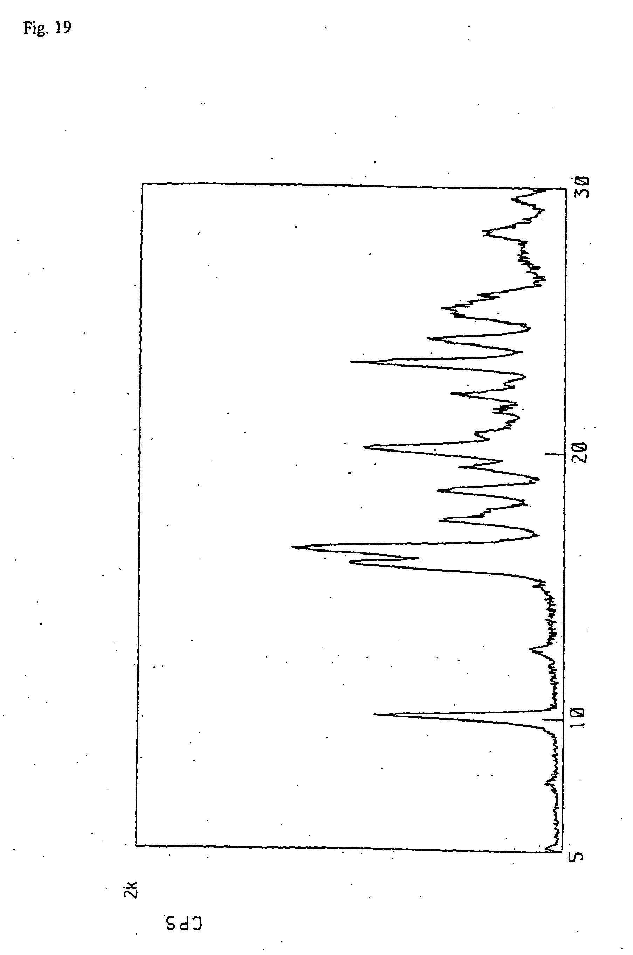

- Fig. 19: is clear powder X-ray diffraction pattern of the polymorph (II).

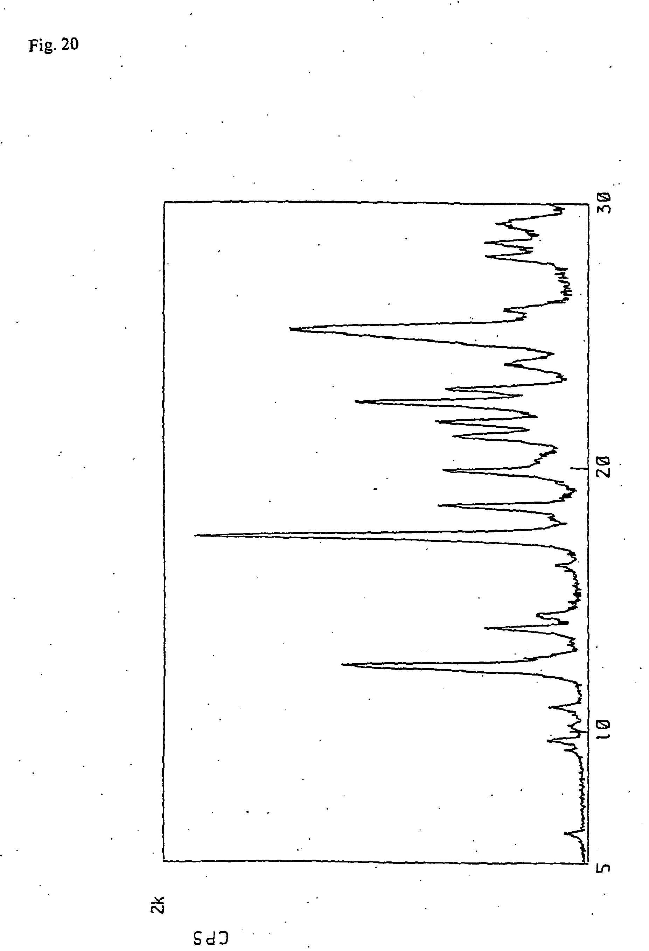

- Fig. 20: is clear powder X-ray diffraction pattern of the polymorph (IV).

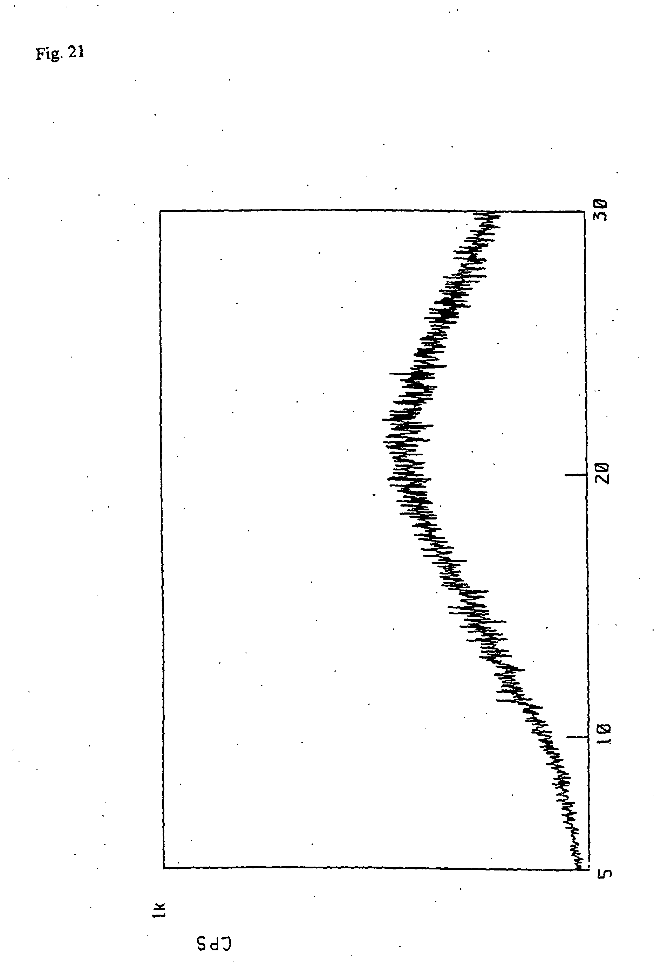

- Fig. 21: is clear powder X-ray diffraction pattern of the Amorphous form.

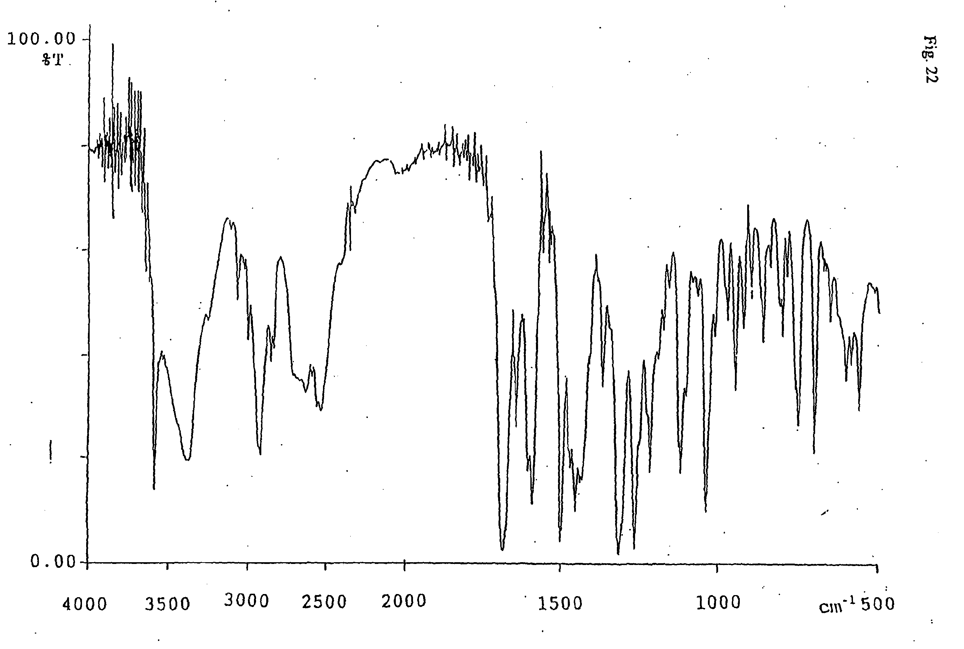

- Fig. 22: is clear infrared absorption in potassium bromide of the polymorph (I).

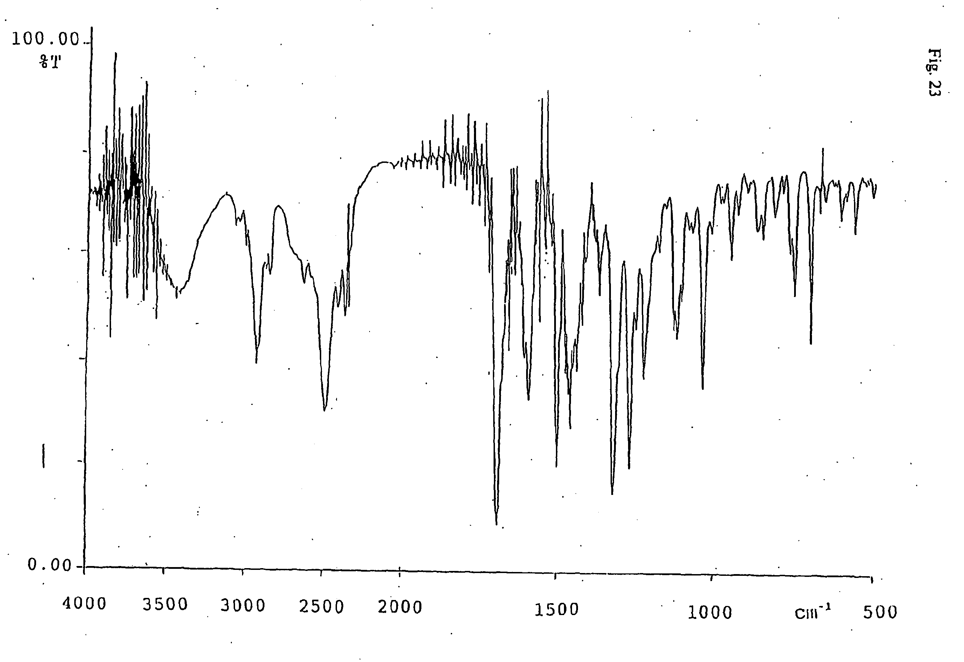

- Fig. 23: is clear infrared absorption in potassium bromide of the polymorph (II).

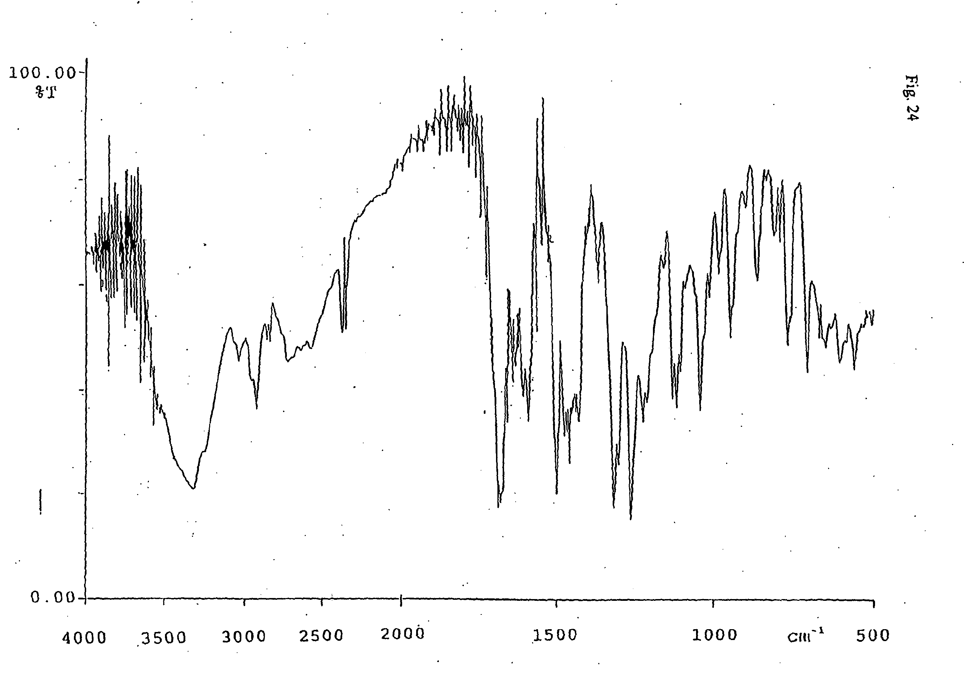

- Fig. 24: is clear infrared absorption in potassium bromide of the polymorph (IV).

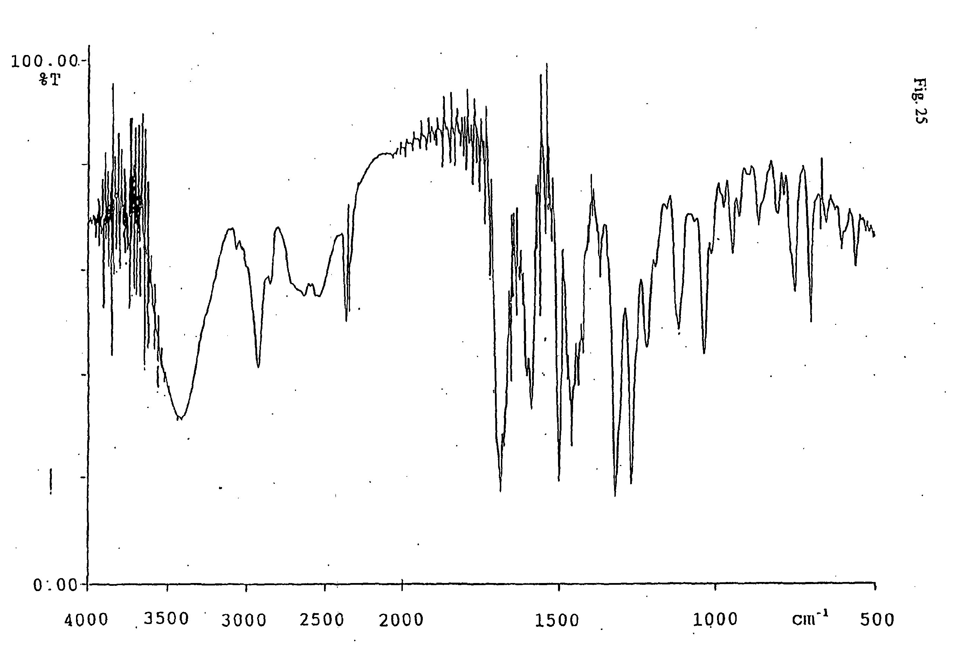

- Fig. 25: is clear infrared absorption in potassium bromide of the amorphous form.

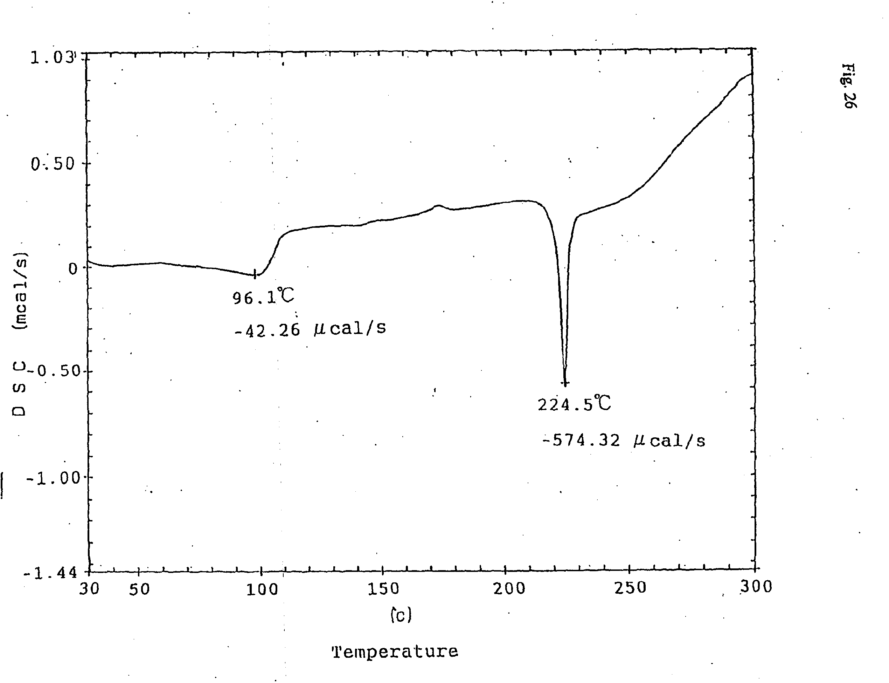

- Fig. 26: is clear thermogravimetric and differential thermal analysis (TG-DTA) of the polymorph (I).

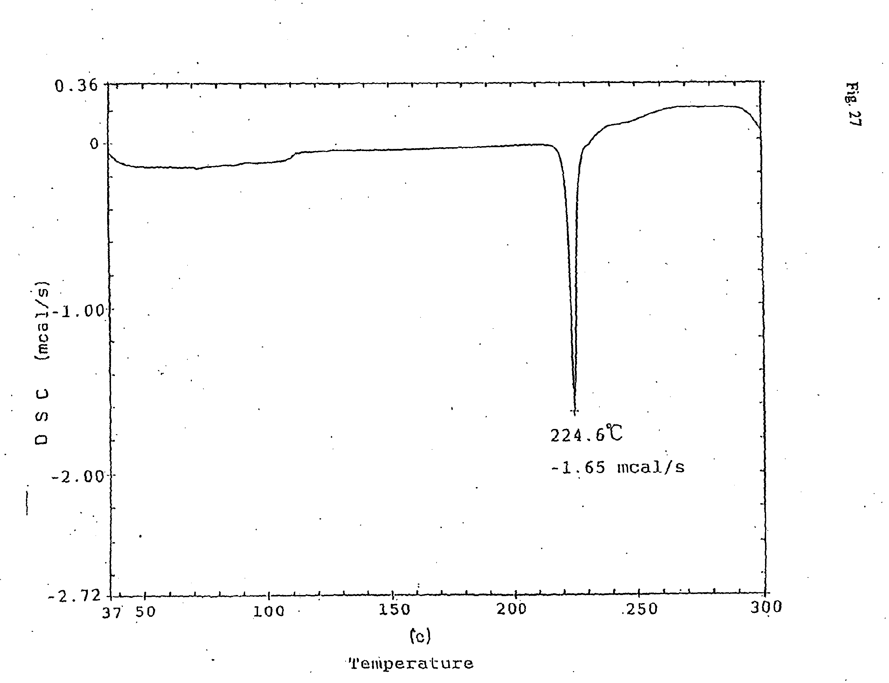

- Fig. 27: is clear thermogravimetric and differential thermal analysis (TG-DTA) of the polymorph (II).

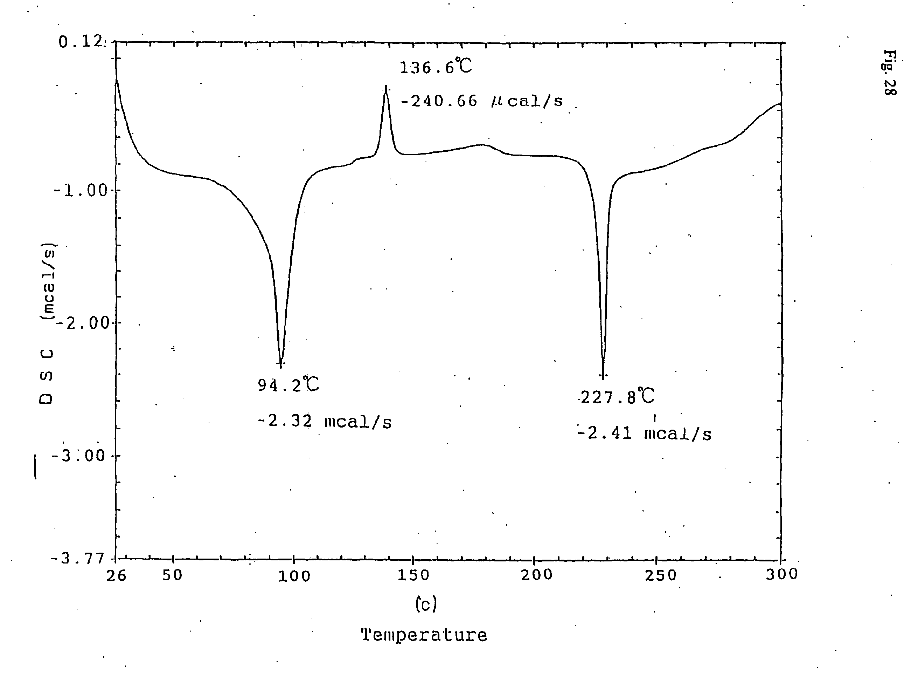

- Fig. 28: is clear thermogravimetric and differential thermal analysis (TG-DTA) of the polymorph (IV).

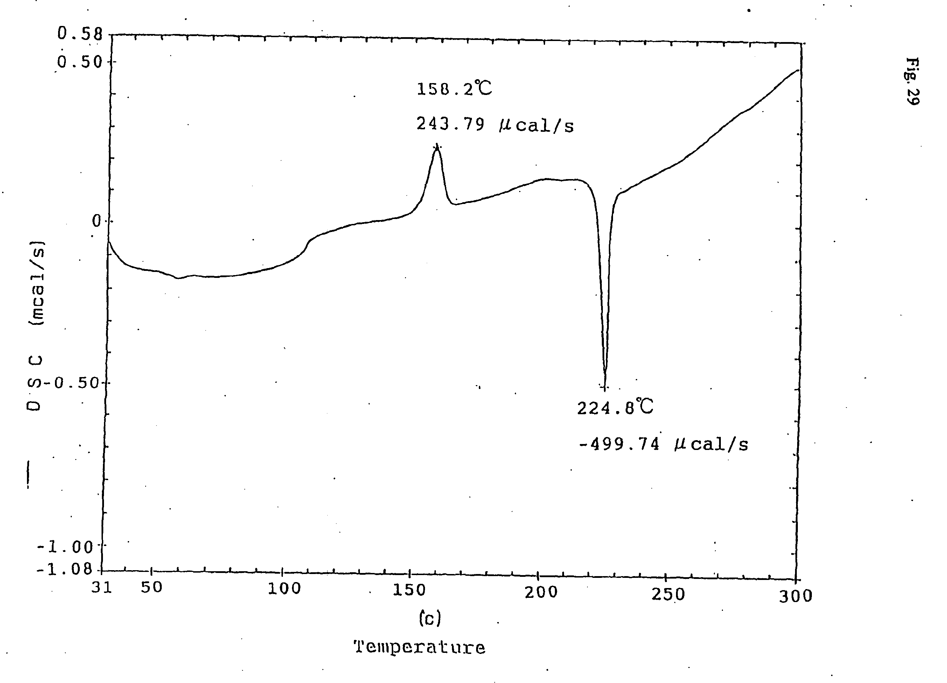

- Fig. 29: is clear thermogravimetric and differential thermal analysis (TG-DTA) of the amorphous form.

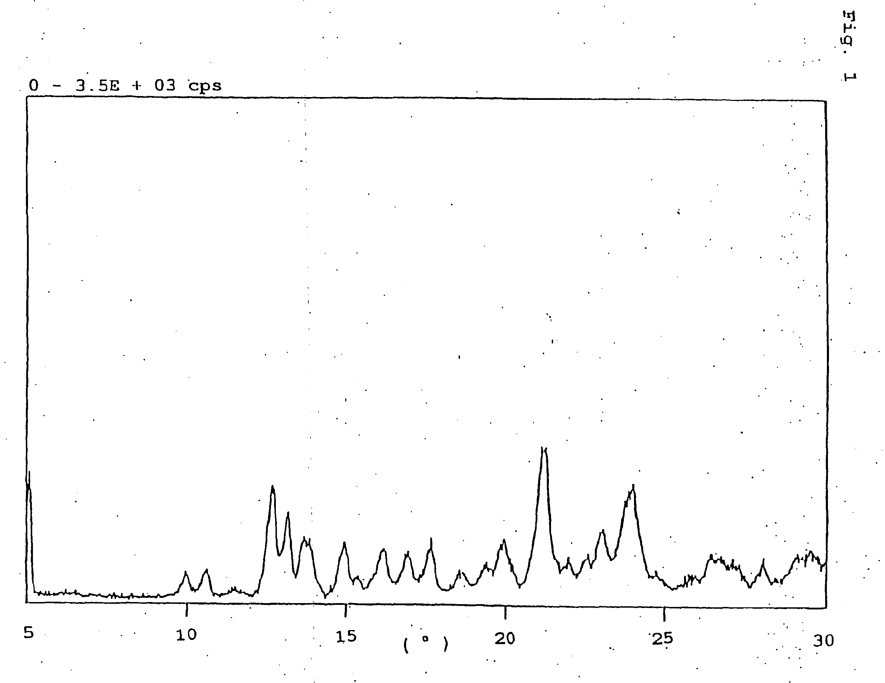

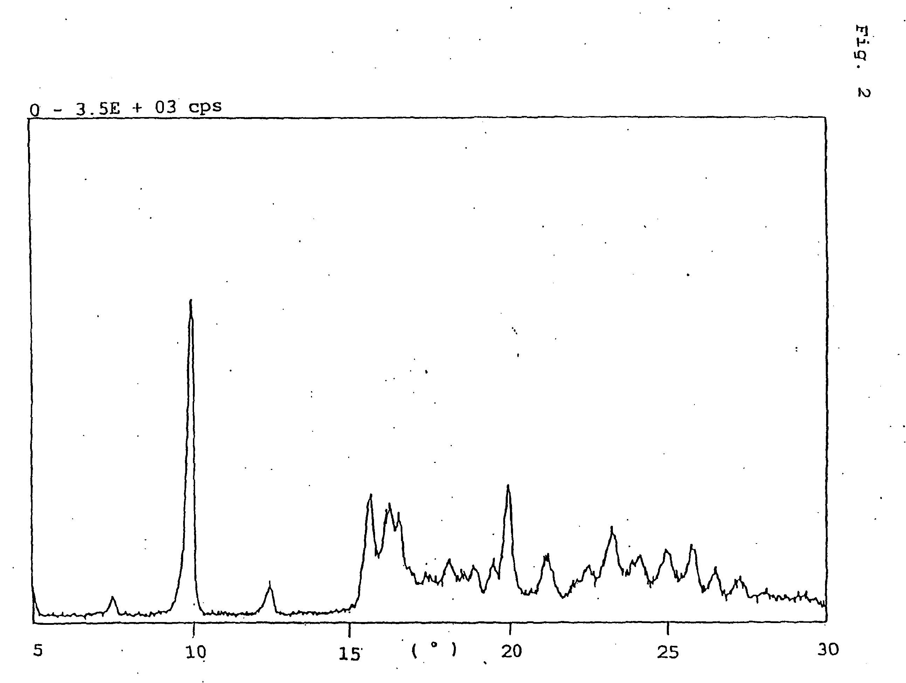

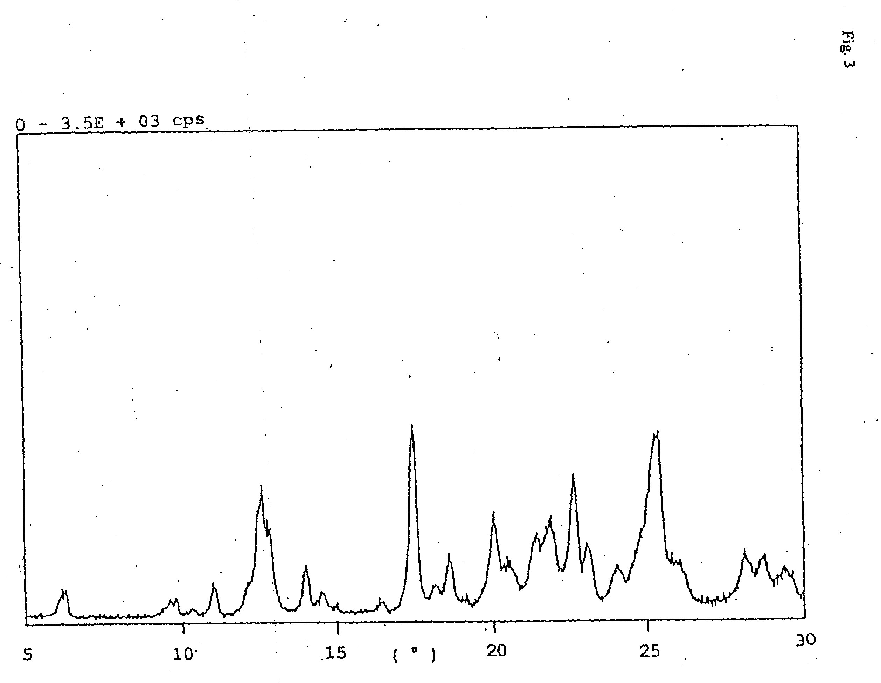

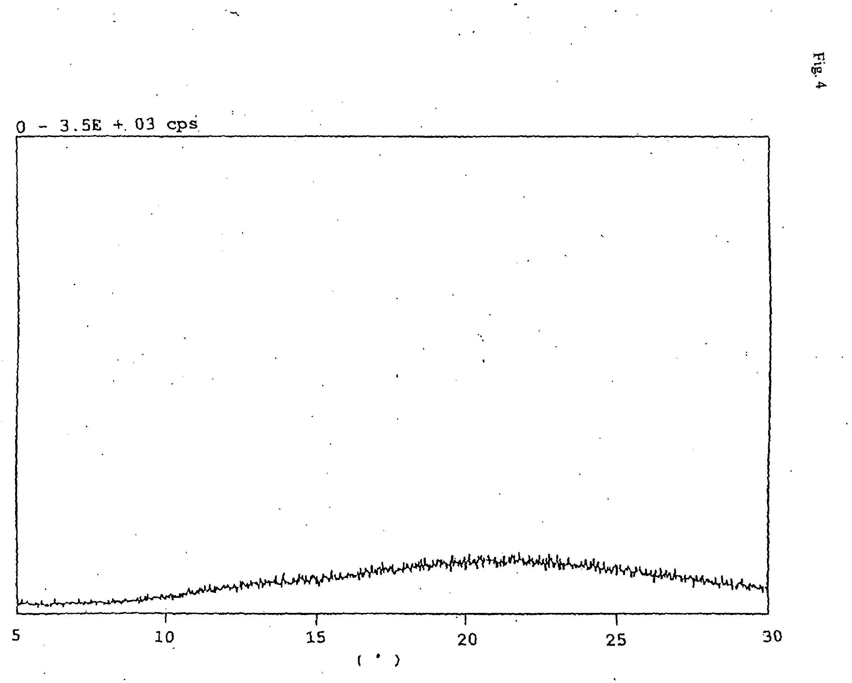

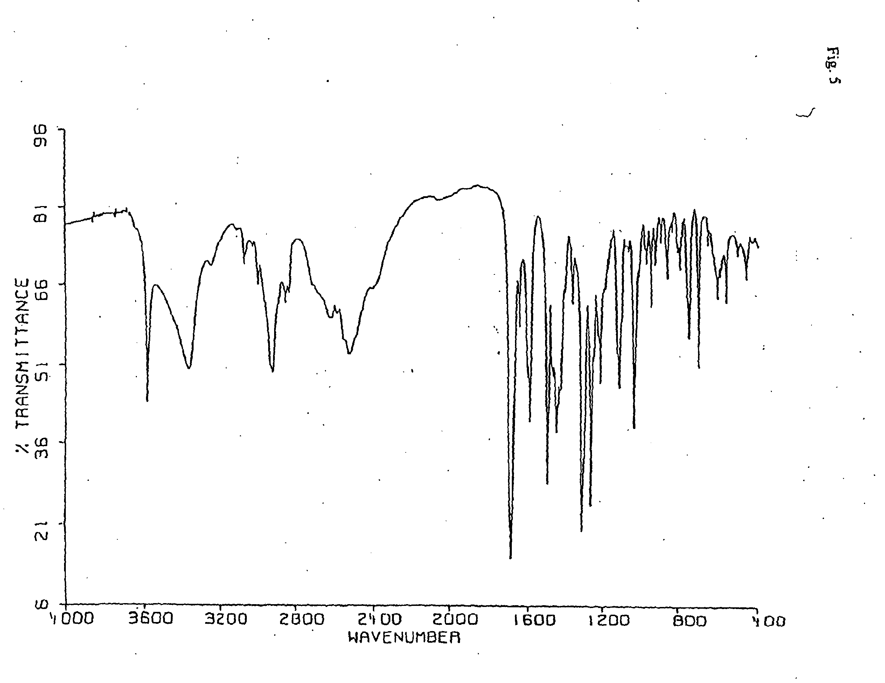

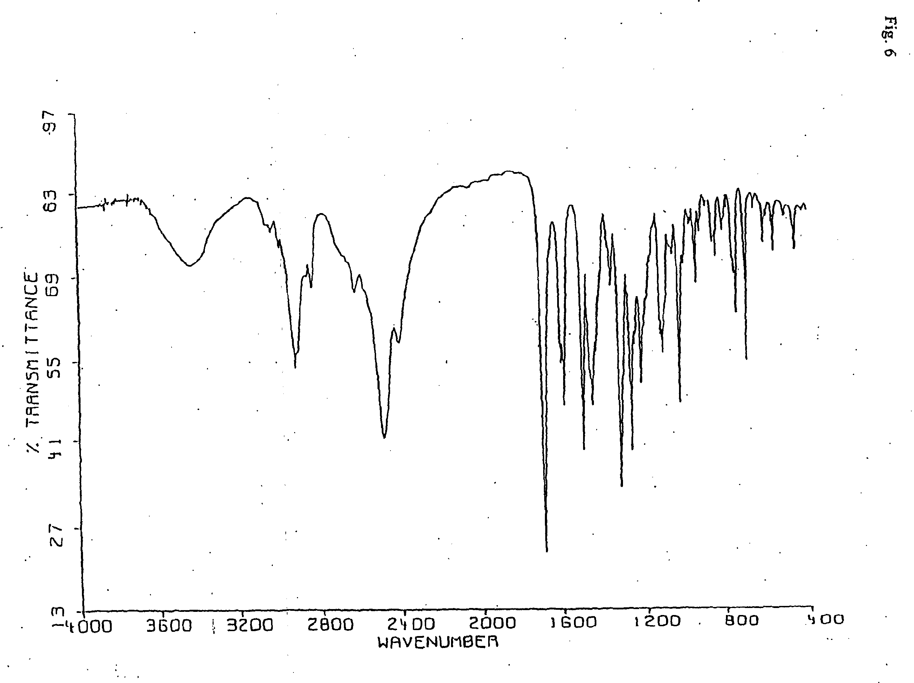

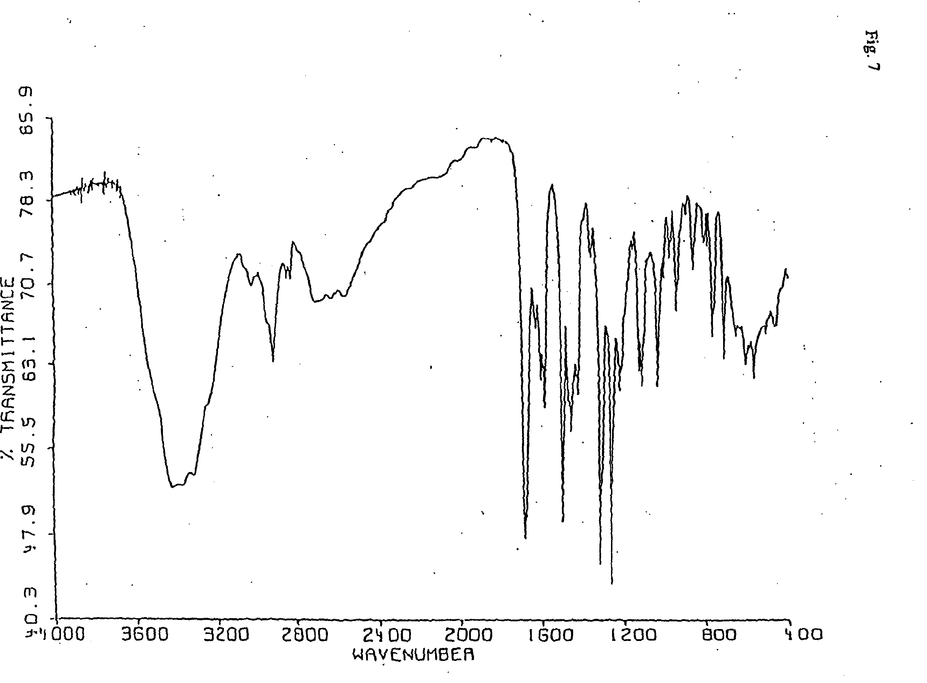

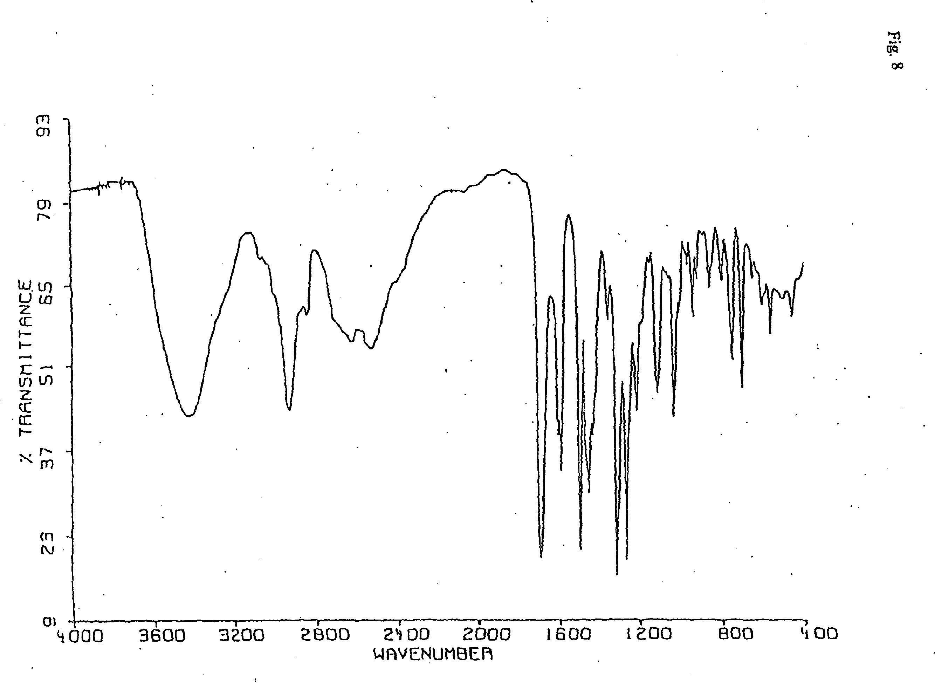

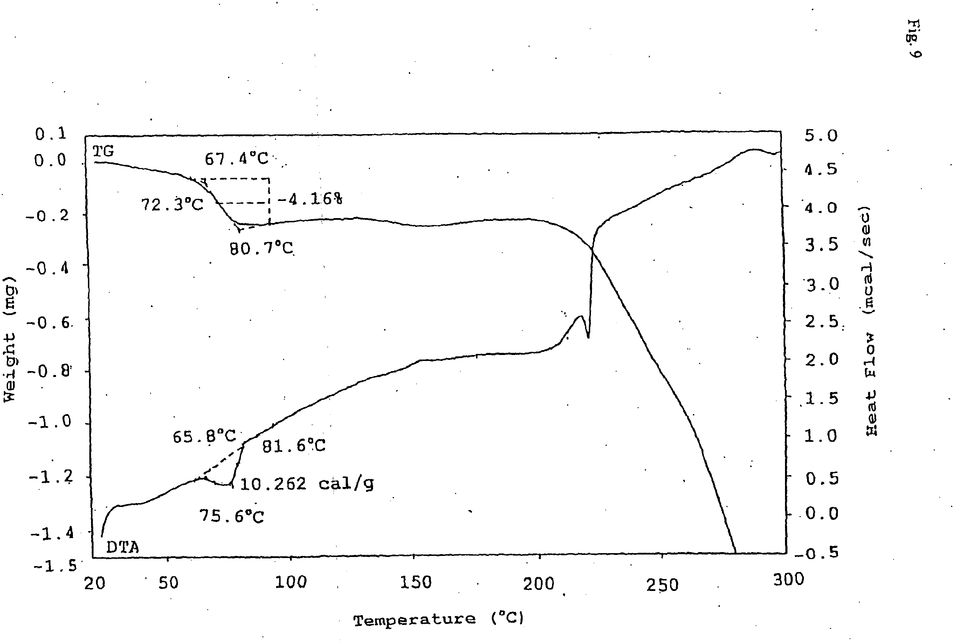

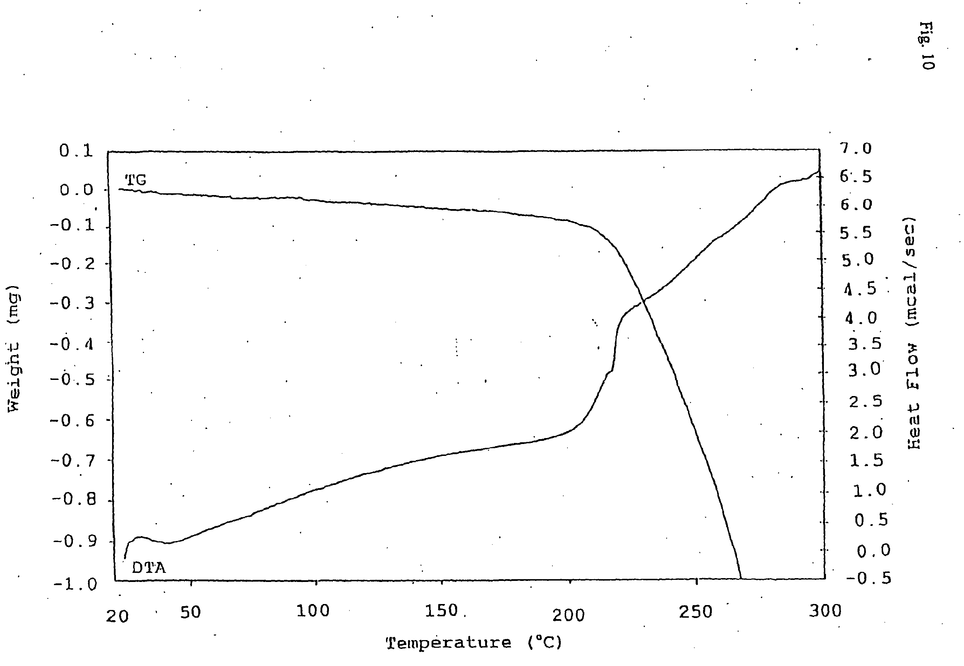

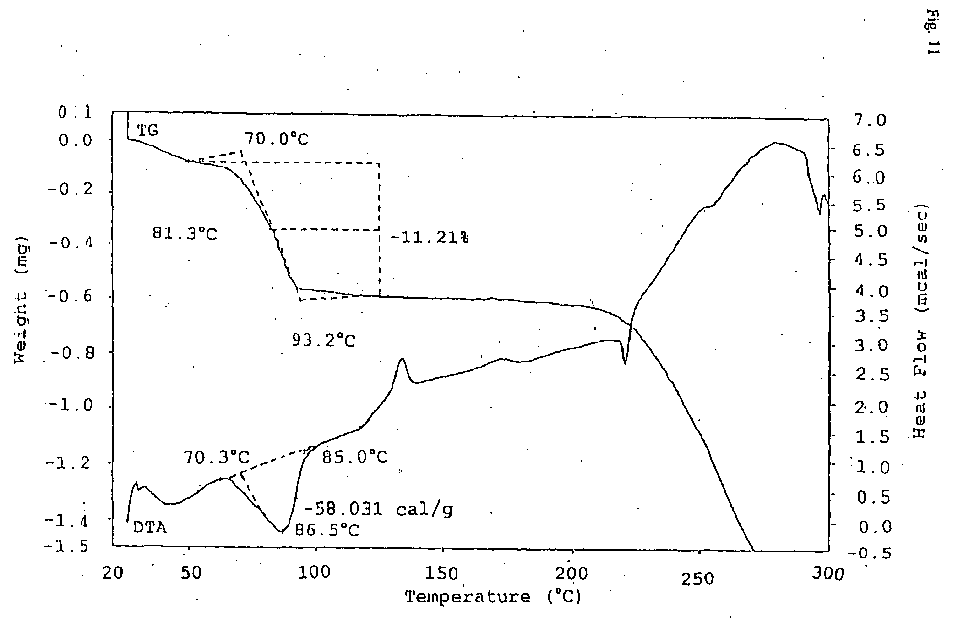

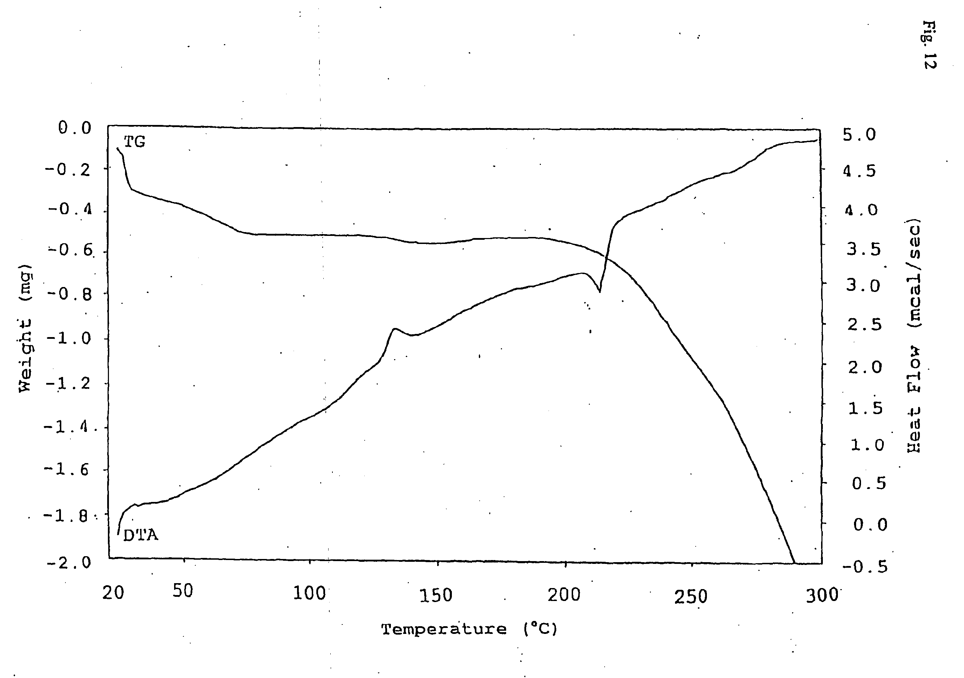

The products shown in Figs. 1 to 12 were again confirmed experimentally to obtain a more clear chart.

### [Examples]

The present invention will now be described in more detail with reference to the following Examples. It is needless to say that the technical scope of the present invention is not limited to these Examples.

### Examples 1 to 8 relates to processes for preparing a polymorph (I).

### Examples 9 to 15, processes for preparing a polymorph (II).

### Examples 16, process for preparing a polymorph (IV).

Reference Examples 1, process for preparing a amorphous form of Donepezil hydrochloride.

### Example 1 (Comparative Example) -; Polymorph (I) of Donepezil hydrochloride

One gram of Donepezil hydrochloride was dissolved in 5 ml of methanol. Adding 10 ml of diisopropyl ether and stirring the mixture in a bath containing ice water, filtration of the separated crystal and drying under atmosphere afforded 0.9 g of the title compound.

### Example 2 (Comparative Example) -; Polymorph (I) of Donepezil hydrochloride

One gram of Donepezil hydrochloride was dissolved in 5 ml of methanol under heating. After cooling to room temperature, 10 ml of isopropyl ether was added. Stirring was continued for 30 minutes at room temperature, then filtration of the separated crystal and drying under atmosphere afforded 0.9 g of the title compound.

### Example 3 (Comparative Example); Polymorph (I) of Donepezil hydrochloride

One gram of Donepezil hydrochloride was dissolved in 5 ml of methanol under heating. After initiation of cooling the solution, it started to separate crystal at 15°C of the inner temperature. After 10. minutes, 10 ml of isopropyl ether was added. Stirring was continued for 1 hour at room temperature, then filtration of the separated crystal and drying under atmosphere afforded 0.9 g of the title compound.

### Example 4 (Comparative Example); Polymorph (I) of Donepezil hydrochloride

5 g of Donepezil hydrochloride was dissolved in 25 ml of methanol under heating, followed by cooling the mixture in a bath containing ice water. Filtration of the separated crystal and drying under atmosphere afforded 4.6 g of the title compound.

### Example 5 (Comparative Example); Polymorph (I) of Donepezil hydrochloride

0.3 g of Donepezil was dissolved in 1.5 ml of methanol, followed by addition of 0.97 ml of 10%-hydrochloric acid in methanol mixture. Filtration of the separated crystal and drying under atmosphere afforded 0.2 g of the title compound.

### Example 6 (Comparative Example); Polymorph (I) of Donepezil hydrochloride

0.3 g of Donepezil was dissolved in 3 ml of ethanol under heating, followed by addition of 3 ml of diisopropyl ether and 0.79 ml of 10%-hydrochloric acid in methanol mixture. Filtration of the separated crystal and drying under atmosphere afforded 0.2 g of the title compound.

### Example 7 (Comparative Example); Polymorph (I) of Donepezil hydrochloride

10 g of Donepezil was dissolved in 100 ml of ethanol under heating. Under stirring, a mixture of concentrated hydrochloric acid (3.1 g) and ethanol (28 ml) was added hereinto, followed by addition of 150 ml of diisopropyl ether. Filtration of the crystals after 10 seconds from the separation and drying under atmosphere afforded 9.36g of the title compound with a yield of 85.4%, a water content of 5.17% and melting point of 225-226° C (Decomposition).

### Example 8 (Comparative Example); Polymorph (I) of Donepezil hydrochloride

10 g of Donepezil hydrochloride was dissolved in 50 ml of methanol under heating. Under stirring in a bath containing ice water, 600 ml of diethyl ether was added. Stirring was continued for 1 hour in the same condition, then filtration of the crystals and drying under atmosphere afforded 10.0 g of the title compound.

### Example 9 (Comparative Example); Polymorph (II) of Donepezil hydrochloride

13.7 g of Donepezil and 4.4ml of hydrochloric acid were dissolved in 100 ml of ethanol under heating. Under stirring at room temperature, 200 ml of diisopropyl ether was added. Filtration of the crystals and drying under vacuum afforded 11.2 g of the title compound.

### Example 10 (Comparative Example); Polymorph (II) of Donepezil hydrochloride

50 g of Donepezil was dissolved in 200 ml of ethanol under heating. After cooling to room temperature, 27.3 g of 18%-hydrogen chloride in ethanol solution was added. After setting calmly for 1 hour, the mixture was concentrated under vacuum, then drying the obtained crystal under atmosphere afforded 55.0 g of the title compound.

### Example 11 (Comparative Example); Polymorph (II) of Donepezil hydrochloride

0.5 g of Donepezil was dissolved in 5 ml of ethanol under heating. Under stirring at room temperature, 1.31 ml of 10%-concentrated hydrochloric acid in ethanol was added, followed by addition of 5 ml of diisopropyl ether. Filtration of the crystals after 10 minutes from the separation and drying under atmosphere afforded 0.4 g of the title compound.

### Example 12 (Comparative Example); Polymorph (II) of Donepezil hydrochloride

5.6 g of Donepezil hydrochloride was dissolved in 30 ml of ethanol, followed by addition of 100 ml of diisopropyl ether. The mixture was cooled in a bath containing ice water. Then filtration of the crystals and drying at 50°C for three days afforded 4.9 g of the title compound.

### Example 13 (Comparative Example); Polymorph (II) of Donepezil hydrochloride

23.3 g of Donepezil hydrochloride was dissolved in 250 ml of ethanol under heating. Under stirring in a bath containing ice water, 600 ml of diethyl ether was added. After setting calmly for 3 hours, filtration of the crystals and drying at 85° C for 22 hours afforded 22.7 g of the title compound.

### Example 14 (Comparative Example); Polymorph (II) of Donepezil hydrochloride

10 g of Donepezil was dissolved in 100 ml of ethanol under heating. Under stirring, 150 ml of a mixture of concentrated hydrochloric acid (3.1 g) and ethanol (28 ml) was added, followed by addition of 150 ml of diisopropyl ether. Filtration of the crystals after 15 minutes from the separation and drying under atmosphere afforded 9.0 g of the title compound with a yield of 82.1% and melting point of 224-226°C (Decomposition).

Example 15 (Comparative Example); Polymorph (II) of Donepezil hydrochloride 40.0 g of Donepezil hydrochloride was dissolved in 700 ml of ethanol under heating. Under cooling in a bath containing ice water, 500 ml of diisopropyl ether was added, and crystallization was done by rubbing the flask wall with spatula.

Then filtration of the crystals and drying at 50° C for 12 hours afforded 31.4 g of the title compound.

### Example 16; Polymorph (IV) of Donepezil hydrochloride

15.0 g of the polymorph (II) of Donepezil hydrochloride was spread on a laboratory dish (Schale) and was continued to stand for 2 weeks under atmosphere having a relative humidity of 100%. 14.8 g of the title compound was obtained with melting point of 226-228°C (Decomposition).

### Reference Example 1; Amorphous form of Donepezil hydrochloride

15.0 g of Donepezil hydrochloride was dissolved in 300 ml of water. The solution was frozen in a bath containing dry ice and acetone and freezing-dried (lyophilizated) for 4 days at -82° C. 14.8 g of the title compound was obtained.

The present invention will be futhermore described in detail with reference to the following Examples.

Examples 17 to 32, processes for preparing a polymorph (I).

Examples 33 to 45 , processes for preparing a polymorph (II).

Examples 46 to 55 relates to processes for preparing a polymorph (IV).

### Example 17 (Comparative Example) Polymorph (I) of Donepezil hydrochloride

1.0 g of Donepezil was dissolved in 4 ml of methanol under heating at 40°C. The solution was cooled in an iced water bath. 0.31 g of Concentrated hydrochloric acid in 1 ml of methanol was added hereinto at 10°C inner temperature. It continued stirring for 90 minutes in an iced water bath. Filtration of the separated crystals followed by drying afforded 0.43 g of the title compound. (water content: 5.33%)

### Example 18 (Comparative Example); Polymorph (I) of Donepezil hydrochloride

1.0 g of Donepezil was dissolved in 4'ml of methanol under heating at 40°C. The solution was cooled in an iced water bath. 0.31 g of Concentrated hydrochloric acid in 1 ml of methanol was added hereinto. After 5 minutes, 30 ml of tert-butyl methyl ether (hereinafter, abbreviated as THYME) was added at 3°C inner temperature. It continued stirring for 30 minutes in an iced water bath. Filtration of the separated crystals followed by drying afforded 1.10 g of the title compound. (water content: 5.60%)

### Example 19 (Comparative Example); Polymorph (I) of Donepezil hydrochloride

1.0 g of Donepezil was dissolved in 4 ml of methanol under heating at 40°C. The solution was cooled in an iced water bath. 0.31 g of Concentrated hydrochloric acid in 1 ml of methanol was added hereinto. After 5 minutes, 30 ml of diisopropyl ether (hereinafter, abbreviated as IPE) was added at 3°C inner temperature. It continued stirring for 30 minutes in an iced water bath. Filtration of the separated crystals followed by drying afforded 1.13 g of the title compound. (water content: 5.50%)

### Example 20 (Comparative Example); Polymorph (I) of Donepezil hydrochloride

1.0 g of Donepezil was dissolved in 4 ml of methanol under Treating at 40°C. The solution was cooled in an iced water bath. 0.31 g of Concentrated hydrochloric acid in 1 ml of methanol was added hereinto at 12°C inner temperature. After 7 minutes, 30 ml of ethyl acetate was added at 3°C inner temperature successively. It continued stirring for 30 minutes in an iced water bath. Filtration of the separated crystals followed by drying afforded 0.71 g of the title compound. (water content: 5.22%)

### Example 21 (Comparative Example); Polymorph (I) of Donepezil hydrochloride

1.0 g of Donepezil was dissolved in 4 ml of ethanol under heating at 40°C. The solution was cooled in an iced water bath. After cooling for 5 minutes, 0.31 g of concentrated hydrochloric acid in 1 ml of ethanol was added hereinto. It continued stirring for 30 minutes in an iced water bath. A small portion of the polymorph (I) of Donepezil hydrochloride was added hereinto. It continued stirring for further 30 minutes in an iced water bath. Filtration of the separated crystals followed by drying afforded 0.70 g of the title compound. (water content: 5.33%)

### Example 22 (Comparative Example); Polymorph (I) of Donepezil hydrochloride

1.0 g of Donepezil was dissolved in 4 ml of tetrahydrofuran (hereinafter, abbreviated as THF) at 24°C. The solution was cooled in an iced water bath. 0.31 g of Concentrated hydrochloric acid in 1 ml of THF was added hereinto. It continued stirring for 40 minutes in an iced water bath. Filtration of the separated crystals followed by drying afforded 1.00 g of the title compound. (water content: 5.67%)

### Example 23 (Comparative Example); Polymorph (I) of Donepezil hydrochloride

1.0 g of Donepezil was dissolved in 9 ml of acetonitrile under heating at 40°C. The solution was cooled in an iced water bath. After cooling for 2 minutes, 0.31 g of concentrated hydrochloric acid in 1 ml of acetonitrile was added hereinto. It continued stirring for 50 minutes in an iced water bath. Filtration of the separated crystals followed by drying afforded 0.63 g of the title compound. (water content: 5.59%)

### Example 24 (Comparative Example); Polymorph (I) of Donepezil hydrochloride

4.0 g of Donepezil was dissolved in 20 ml of methanol under heating at 40°C. The solution was cooled in an iced water bath. Hydrogen chloride gas was blown hereinto at 3°C inner temperature, until the atmosphere turns acidic. It continued stirring for 20 minutes in an iced water bath. Filtration of the separated crystals followed by drying afforded 3.40 g of the title compound. (water content: 5.19%)

### Example 25 (Comparative Example); Polymorph (I) of Donepezil hydrochloride

10.0 g of Donepezil hydrochloride was dissolved in 60 ml of methanol under reflux. Heating was ceased. 120 ml of IPE was added hereinto at 60°C inner temperature. It continued stirring for 20 minutes in an iced water bath. Filtration of the separated crystals followed by drying afforded 9.80 g of the title compound. (water content: 5.87%).

### Example 26 (Comparative Example); Polymorph (I) of Donepezil hydrochloride

3.0 g of Donepezil hydrochloride was dissolved in 18 ml of methanol under reflux. Heating was ceased. 36 ml of IPE was added hereinto at 54°C inner temperature. It continued stirring for 20 minutes in an iced water bath. Filtration of the separated crystals followed by drying afforded 2.95 g of the title compound. (water content: 5.55%)

| Elementary Analysis: | | | | |
|---|---|---|---|---|
| | C | H | N | Cl |
| found(%) | 65.55 | 7.53 | 3.05 | 8.16 |

### Example 27 (Comparative Example); Polymorph (I) of Donepezil hydrochloride

1.0 g of Donepezil hydrochloride was dissolved in 5 ml of methanol under reflux. The solution was cooled in an iced water bath. 30 ml of TBME was added hereinto at 3°C inner temperature. It continued stirring for 30 minutes in an iced water bath. Filtration of the separated crystals followed by drying afforded 1.00 g of the title compound. (water content: 5.40%)

### Example 28 (Comparative Example); Polymorph (I) of Donepezil, hydrochloride

1.0 g of Donepezil hydrochloride was dissolved in 5 ml of methanol under reflux. The solution was cooled in an iced water bath. After cooling for 2 minutes, it started to separate crystals. 1 Minutes passed from the initiation of separation, 30 ml of ethyl acetate was added hereinto. It continued stirring for 30 minutes in an iced water bath. Filtration of the separated crystals followed by drying afforded 1.00 g of the title compound. (water content: 5.60%)

### Example 29 (Comparative Example); Polymorph (I) of Donepezil hydrochloride

1.0 g of Donepezil hydrochloride was dissolved in 5 ml of methanol under reflux. The solution was cooled in an iced water bath. 30 ml of n-hexane was added hereinto at 3°C inner temperature. It continued stirring for 70 minutes in an iced water bath. Filtration of the separated crystals followed by drying afforded 0.89 g of the title compound. (water content: 5.66%)

### Example 30 (Comparative Example); Polymorph (I) of Donepezil hydrochloride

1.0 g of Donepezil hydrochloride was dissolved in 20 ml of ethanol under reflux. The solution was cooled in an iced water bath. It continued stirring for 70 minutes in an iced water bath. Filtration of the separated crystals followed by drying afforded 0.48 g of the title compound. (water content: 5.72%)

### Example 31 (Comparative Example); Polymorph (I) of Donepezil hydrochloride

1.0 g of Donepezil hydrochloride was dissolved in 25 ml of ethanol under reflux. The solution was cooled in an iced water bath. 50 ml of IPE was added at 3°C inner temperature. It continued stirring for 5 minutes in an iced water bath. Filtration of the separated crystals followed by drying afforded 0.86 g of the title compound. (water content: 5.32%)

### Example 32 (Comparative Example); Polymorph (I) of Donepezil hydrochloride

1.0 g of Donepezil hydrochloride was dissolved in 20 ml of ethanol under reflux. The solution was cooled in an iced water bath. 30 ml of TBME was added hereinto at 3°C inner temperature. It continued stirring for 30 minutes in an iced water bath. Filtration of the separated crystals followed by drying afforded 1.00 g of the title compound. (water content: 5.33%)

### Example 33 (Comparative Example); Polymorph (II) of Donepezil hydrochloride

1.0 g of Donepezil was dissolved in 4 ml of ethanol under heating at 40°C. The solution was cooled in an iced water bath. After cooling for 4 minutes, 0.31 g of concentrated hydrochloric acid in 1 ml of ethanol was added hereinto. After 3 minutes, 30 ml of TBME was added hereinto. It continued stirring for 50 minutes in an iced water bath. Filtration of the separated crystals followed by drying afforded 1. 08 g of the title compound (water content: 1.78%)

### Example 34 (Comparative Example); Polymorph (II) of Donepezil hydrochloride

1.0 g of Donepezil was dissolved in 9 ml of isopropyl alcohol (hereinafter, abbreviated as IPA) under heating at 40°C. The solution was cooled in an iced water bath. 0.31 g of Concentrated hydrochloric acid in 1 ml of IPA was added hereinto. It continued stirring for 30 minutes in an iced water bath. Filtration of the separated crystals followed by drying afforded 0.87 g of the title compound. (water content: 1.10%)

### Example 35 (Comparative Example); Polymorph (II) of Donepezil hydrochloride

1.0 g of Donepezil was dissolved in 9 ml of acetone at 19°C. 0.31 g of Concentrated hydrochloric acid in 1 ml of acetone was added hereinto. It continued stirring for 5 minutes at room temperature. Filtration of the separated crystals followed by drying afforded 0.87 g of the title compound. (water content: 0.83%)

### Example 36 (Comparative Example): Polymorph (II) of Donepezil hydrochloride

1.0 g of Donepezil was dissolved in 9 ml of acetone at 24°C. 0.31 g of Concentrated hydrochloric acid in 1 ml of acetone was added hereinto. It continued stirring for 30 minutes in an iced water bath. Filtration of the separated crystals followed by drying afforded 0. 92 g of the title compound. (water content: 0.61%)

### Example 37 (Comparative Example); polymorph (II) of Donepezil hydrochloride

1.0 g of Donepezil was dissolved in 9 ml of THF at 24°C. 0.31 g of Concentrated hydrochloric acid in 1 ml of THF was added hereinto. It continued stirring for 30 minutes in an iced water bath. Filtration of the separated crystals followed by drying afforded 1.09 g of the title compound. (water content: 0.78%)

### Example 38 (Comparative Example); Polymorph (II) of Donepezil hydrochloride

3.0 g of Donepezil was dissolved in 30 ml of acetone at 21°C. Hydrogen chloride gas was blown hereinto at room temperature, until the atmosphere turns acidic. After stirring for 3 minutes, filtration of the separated crystals followed by drying afforded 2.80 g of the title compound. (water content: 2.78%)

### Example 39 (Comparative Example); Polymorph (II) of Donepezil hydrochloride

4.0 g of Donepezil was dissolved in 20 ml of methylene chloride at 18°C. The solution was cooled in an iced water bath. Hydrogen chloride gas was blown hereinto at 4°C inner temperature, until the atmosphere turns acidic. Argon gas was blown hereinto. After stirring for 2 hours in an iced water bath, filtration of the separated crystals followed by drying afforded 4.09 g of the title compound. (water content: 0.81%)

### Example 40 (Comparative Example); Polymorph (II) of Donepezil hydrochloride

1.0 g of Donepezil hydrochloride was dissolved in 20 ml of ethanol under reflux. The solution was cooled in an iced water bath. 30 ml of TBME was added hereinto at 20°C inner temperature. It continued stirring for 3 hours in an iced water bath. Filtration of the separated crystals followed by drying afforded 0.92 g of the title compound. (water content: 0.79%)

### Example 41 (Comparative Example); Polymorph (II) of Donepezil hydrochloride.

1.0 g of Donepezil hydrochloride was dissolved in 20 ml of ethanol under reflux. The solution was cooled to room temperature in an iced water bath. 30 ml of TBME was added hereinto at 20°C inner temperature. It continued stirring for 20 minutes at room temperature. Filtration of the separated crystals followed by drying afforded 0.80 g of the title compound. (water content: 0.52%)

### Example 42 (Comparative Example); Polymorph (II) of Donepezil hydrochloride

10.0 g of Donepezil hydrochloride was dissolved in 100 ml of ethanol under reflux. Under stirring, the solution was added into 200 ml of IPE cooled in an iced water bath. It continued stirring for 5 minutes Filtration of the separated crystals followed by drying afforded 9. 40 g of the title compound. (water content: 0.19%)

| Elementary Analysis: | | | | |
|---|---|---|---|---|
| | C | H | N | Cl |
| found(%) | 69.12 | 7.20 | 3.32 | 8.61 |

### Example 43 (Comparative Example); polymorph (II) of Donepezil hydrochloride

1.0 g of Donepezil hydrochloride was dissolved in 20 ml of methylene chloride at 18°C. The solution was cooled in an iced water bath. 30 ml of TBME was added hereinto. After stirring for 3 minutes in an iced water bath, filtration of the separated crystals followed by drying afforded 0.88 g of the title compound. (water content: 2.33%.)

### Example 44 (Comparative Example); Polymorph (II) of Donepezil hydrochloride

2.0 g of The polymorph (I) of Donepezil hydrochloride was spread on an laboratory dish (Schale) and was allowed to stand for 16 hours under reduced pressure at 80°C. 1. 89 g of the title compound was obtained. (water content: 0.22%)

### Example 45 (Comparative Example); Polymorph (II) of Donepezil hydrochloride

2.0 g of The amorphous form of Donepezil hydrochloride was spread on an laboratory dish (Schale) and was allowed to stand for 16 hours under reduced pressure at 80°C. 1.98 g of the title compound was obtained. (water content: 1.15%) was added hereinto at 40°C inner temperature. It continued stirring for 20 minutes at 60°C inner temperature. Filtration of the separated crystals followed by drying afforded the title compound.

### Example 46; Polymorph (IV) of Donepezil hydrochloride

2.0 g of Donepezil was dissolved in the mixture comprising from 0.65 g of concentrated hydrochloric acid and 10 ml of ion exchange purified water under stirring at 22°C. After stirring at room temperature for 1 hour, filtration of the separated crystals followed by drying afforded 1.80 g of the title compound. (water content: 11.00%)

### Example 47; Polymorph (IV) of Donepezil hydrochloride

1.0 g of Donepezil was dissolved in the mixture comprising from 0.31 g of concentrated hydrochloric acid and 4 ml of ion exchange purified water under stirring at 22°C. 100 ml of THF was added hereinto. It continued stirring for 30 minutes at room temperature. Filtration of the separated crystals followed by drying afforded 1.06 g of the title compound. (water content: 11.14%)

### Example 48 ; Polymorph (IV) of Donepezil hydrochloride

3.0 g of Donepezil was dissolved in the mixture comprising from 88 ml of THF and 3 ml of ion exchange purified water under stirring at 20°C. 0.93 g of Concentrated hydrochloric acid in 2 ml of THF was added hereinto. After stirring for 30 minutes in an iced water bath, filtration of the separated crystals followed by drying afforded 3.21 g of the title compound. (water content: 11.34%)

| Elementary Analysis: | | | | |
|---|---|---|---|---|
| | C | H | N | Cl |
| found(%) | 61.30 | 7.76 | 2.86 | 7.68 |

### Example 49; Polymorph (IV) of Donepezil hydrochloride

1.0 g of Donepezil was dissolved in 9 ml of toluene at 22°C. 0.31 g of Concentrated hydrochloric acid in 1 ml of toluene was added hereinto. It continued stirring over night at room temperature. Filtration of the separated crystals followed by drying afforded 1.23 g of the title compound. (water content: 11.40%)

### Example 50 ; Polymorph (IV) of Donepezil hydrochloride

1.0 g of Donepezil was dissolved in 9 ml of n-hexane at 21°C. 0.31 g of Concentrated hydrochloric acid in 1 ml of n-hexane was added hereinto. It continued stirring over night at room temperature. Filtration of the separated crystals followed by drying afforded 1.23 g of the title compound. (water content: 11.24%)

### Example 51 , Polymorph (IV) of Donepezil hydrochloride

1.0 g of Donepezil was dissolved in the mixture comprising from 1 ml of methanol, 3 ml of ion exchange purified water and 0.93g of concentrated hydrochloric acid at 20°C. It continued stirring for 3 days at room temperature. Filtration of the separated crystals followed by drying afforded 0.83 g of the title compound. (water content: 11.04%)

### Example 52 ; Polymorph (IV) of Donepezil hydrochloride

2. 0 g of Donepezil was suspended in 10 ml of ion exchange purified water. Hydrogen chloride gas was blown hereinto at 23°C inner temperature, until it became homogeneous. After stirring for 2.5 hours at room temperature, filtration of the separated crystals followed by drying afforded 1.77 g of the title compound. (water content: 11.10%)

### Example 53 ; Polymorph (IV) of Donepezil hydrochloride

1.0 g of Donepezil hydrochloride was dissolved in 5 ml of ion exchange purified water under heating at 60°C. The solution was cooled to room temperature. After stirring over night at room temperature, filtration of the separated crystals followed by drying afforded 0.92 g of the title compound. (water content: 11.12%)

### Example 54 ; Polymorph (IV) of Donepezil hydrochloride

1.0 g of The polymorph (II) of Donepezil hydrochloride was spread on an laboratory dish (Schale) and was allowed to stand for 24 hours under atmosphere having an relative humidity of 90%. 1.15 g of the Polymorph (IV) was obtained. (water content: 12.33%)

### Example 55 ; Amorphous Form & Polymorph (IV) of Donepezil hydrochloride

10.0 g of the polymorph (III) of Donepezil hydrochloride was spread on an laboratory dish (Schale, φ=250mm), and was dissolved in 300 ml of ion exchange purified water at 21°C. This solution was moved into freeze-drying apparatus, and dried for 3 days to afford 9.90 g of amorphous form. This material was kept standing for 24 hours under atmosphere having an relative humidity of 90%. 11.20 g of the Polymorph (IV) was obtained. (water content: 11.21%)

Finally, the efficacy of the present invention in view of the stability or hygroscopicity will now be described in comparison with amorphous form of Donepezil hydrochloride. The invention provides advantageous results as follows:

### (1) Stability Assay

### (Method for measurement)

10mg of each of the polymorphs (I), (II) and (IV) of Donepezil hydrochloride was taken as a couple of samples into tubes, respectively. They were stored under the hollowing conditions and impurity's contents were measured periodically.

| Condition | Storage Period | | | |
|---|---|---|---|---|
| 80° C | 1 week | 2 weeks | | |
| 60° C | | 2 weeks | 1 month | |
| 40°C | | | 1 month | 3 months |
| -20°C | 1 week | 2 weeks | 1 month | 3 months |

### (Method and condition for measurement of HPLC purity)

10ml of the following mobile phase for HPLC was added into each tube of the aforementioned samples. Then impurity's contents were measured for each sample under the following conditions.

The average was calculated from two results.

| | |
|---|---|
| Column (Solid phase) | ;Inertsil ODS-II (4.6 mm I.D.×150 mm) |
| Mobile Phase | ; CH₃CN/water/70% HClO₄ |
| | (V/V/V=300:700:1) |
| Detector | ; UV271 nm |
| Flow rate | ; 1.0 ml/min. |
| Injection Volume | ; 5 ml |
| Column Temperature | ; room temperature |

### Results:

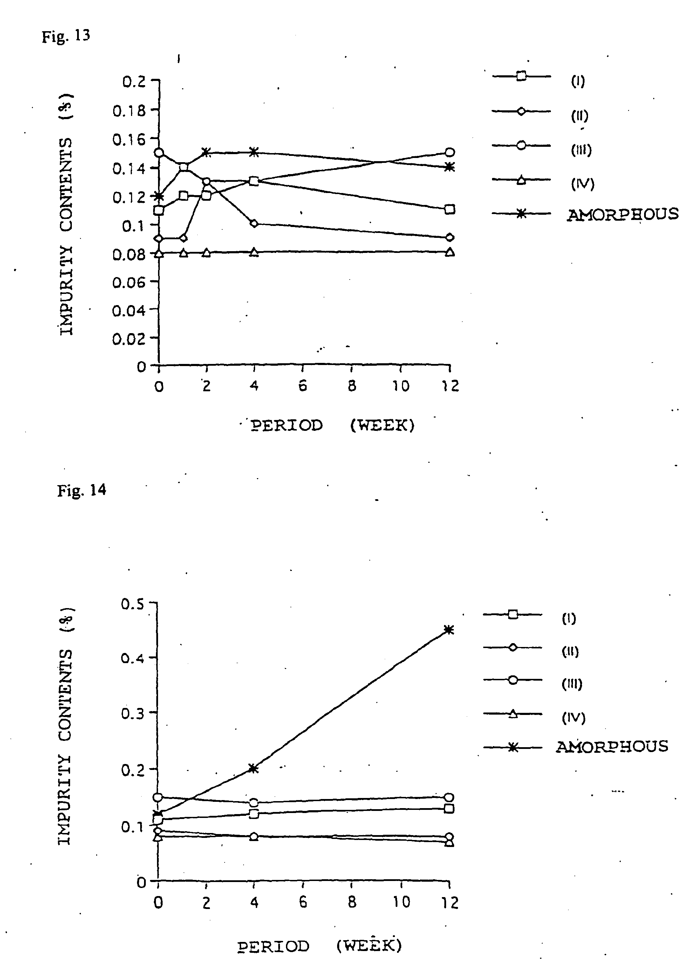

### 1) Storage at -20° C. (refer to Fig. 13)

| Impurity(%) months | 0 | 1 week | 2 weeks | 1 month | 3 |
|---|---|---|---|---|---|
| Polymorph (I) | 0.11 | 0.12 | 0.12 | 0.13 | 0.11 |
| Polymorph (II) | 0.09 | 0.09 | 0.13 | 0.10 | 0.09 |
| | | | | | |
| Polymorph (IV) | 0.08 | 0.08 | 0.08 | 0.08 | 0.08 |
| Amorphous | 0.12 | 0.14 | 0.15 | 0.15, | 0.14 |

### 2) Storage at 40° C. (refer to Fig. 14)

| Impurity(%) | 0 | 1 month | 3 months |
|---|---|---|---|
| Polymorph (I) | 0.11 | 0.12 | 0.13 |
| Polymorph (II) | 0.09 | 0.08 | 0.08 |
| | | | |
| Polymorph (IV) | 0.08 | 0.08 | 0.07 |
| Amorphous | 0.12 | 0.20 | 0.45 |

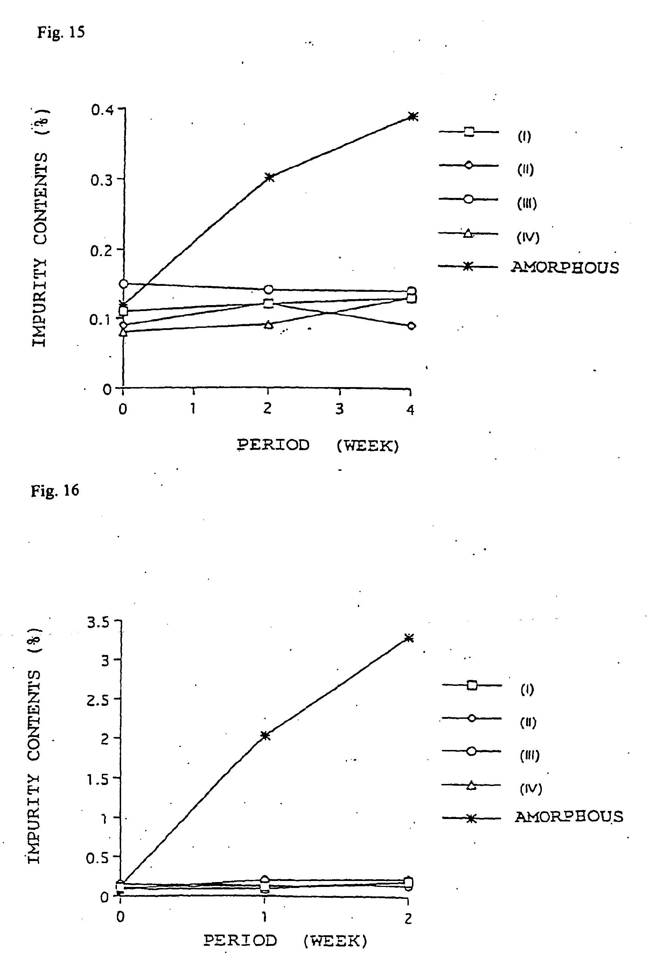

### 3) Storage at 60° C. (refer to Fig. 15)

| Impurity(%) | 0 | 2 weeks | 1 month |
|---|---|---|---|
| Polymorph (I) | 0.11 | 0.12 | 0.13 |
| Polymorph (II) | 0.09 | 0.12 | 0.09 |
| | | | |
| Polymorph (IV) | 0.08 | 0.09 | 0.13 |
| Amorphous | 0.12 | 0.30 | 0.39 |

### 4) Storage at 80° C. (refer to Fig. 16)

| Impurity(%) | 0 | 1 week | 2 weeks |
|---|---|---|---|
| Polymorph (I) | 0.11 | 0.12 | 0.19 |
| Polymorph (II) | 0.09 | 0.20 | 0.22 |
| | | | |
| Polymorph (IV) | 0.08 | 0.09 | 0.19 |
| Amorphous | 0.12 | 2.02 | 3.29 |

It is evident from the above results that the polymorphs (I), (II) and (IV) is superior in stability against heat to the amorphous form.

### (2) Hygroscopicity Assay

### (Method for measurement)

Polymorphs (I), (II) and (IV) were stored under atmosphere having the following relative humidities at 25°C. Water contents were measured according to the general method (Karl Fischer Method) introduced by the Japanese Pharmacopoeia.

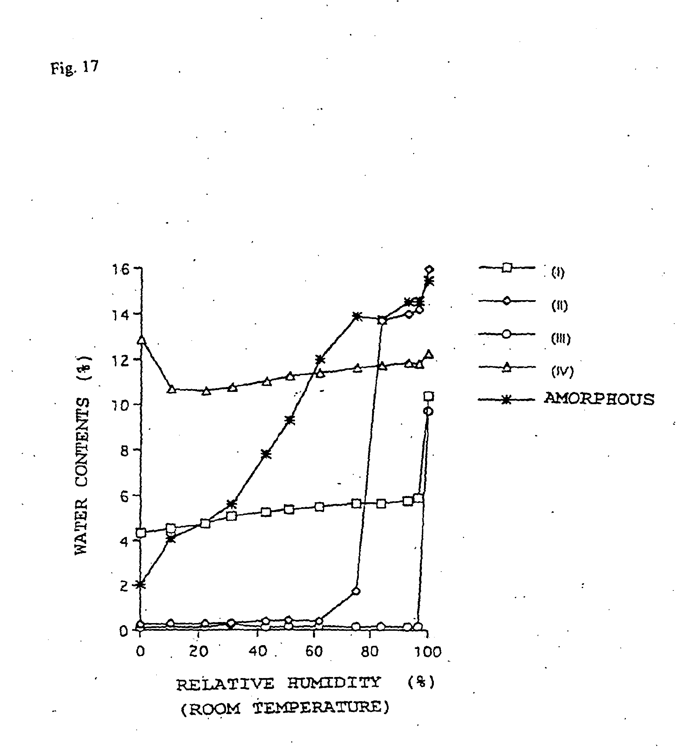

### Results (refer to Fig. 17)

| | | Water contents(%) Polymorph | | |
|---|---|---|---|---|
| Relative Humidity | | | | |
| (%) | (I) | (II) | (IV) | Amorphous |
| Initiation | 4.34 | 0.26 | 12.87 | 2.03 |
| 10.6 | 4.54 | 0.28 | 10.70 | 4.09 |
| 22.2 | 4.75 | 0.29 | 10.60 | 4.78 |
| 31.0 | 5.07 | 0.32 | 10.77 | 5.61 |
| 42.8 | 5.25 | 0.39 | 11.03 | 7.80 |
| 51.0 | 5.38 | 0.43 | 11.28 | 9.29 |
| 61.8 | 5.49 | 0.40 | 11.40 | 12.01 |
| 75.0 | 5.65 | 1.73 | 11.62 | 13.89 |
| 84.0 | 5.64 | 13.70 | 11.72 | 13.74 |
| 93.0 | 5.76 | 13.99 | 11.84 | 14.51 |
| 96.6 | 5.88 | 14.18 | 11.80 | 14.53 |
| 100.0 | 10.37 | 15.93 | 12.26 | 15.44 |

In the above results, the polymorphs (I), (II) and (IV) did not show hygroscopicity until a relative humidity of 96.6%, until 75.0%, until 100%, respectively. The amorphous Donepezil hydrochloride showed hygroscopicity at and thereafter 10.6%. Those experimental results show that the polymorphs of Donepezil hydrochloride (I), (II) and (IV) have an excellent heat stability and a low hygroscopicity.

## Claims

1. Donepezil hydrochloride, 1-benzyl-4-[(5,6-dimethoxy-1-indanon)-2-yl]methylpiperidine hydrochloride, in the form of polymorph (IV), the polymorph being specified by peaks at below shown diffraction degrees with the below shown intensity in terms of I/I₀ in powder X-ray diffraction pattern and the below shown absorption peaks in infrared absorption spectra in potassium bromide in terms of reciprocal centimeters:
Polymorphs (IV)
Peaks in the powder X-ray Diffraction pattern are:
| Diffraction angles | | Intensity |
|---|---|---|
| (2θ,°) | (I/Iₒ) | |
| 6.24 | | 15 |
| 9.66 | | 12 |
| 11.04 | | 22 |
| 12.12 | | 24 |
| 12.54 | | 67 |
| 12.76 | | 61 |
| 13.98 | | 27 |
| 14.42 | | 15 |
| 14.88 | | 11 |
| 16.34 | | 12 |
| 17.46 | | 100 |
| 18.12 | | 25 |
| 18.60 | | 32 |
| 19.06 | | 15 |
| 19.98 | | 74 |
| 20.42 | | 41 |
| 20.62 | | 34 |
| 21.30 | | 48 |
| 21.80 | | 63 |
| 22.58 | | 78 |
| 23.04 | | 46 |
| 24.00 | | 32 |
| 24.54 | | 49 |
| 25.14 | | 90 |
| 25.36 | | 99 |
| 26.06 | | 34 |
| 28.10 | | 41 |
| 28.58 | | 39 |
| 29.30 | | 31 |
| 29.44 | | 28 |
Wave numbers (cm⁻¹) of Infrared absorption spectra in potassium bromide are:
401, 431, 459, 467, 490, 506, 518, 561, 586, 606, 631, 651, 709, 753, 766, 857, 944, 1009, 1041, 1106, 1119, 1132, 1213, 1225, 1265, 1304, 1318, 1429, 1458, 1470, 1500, 1589, 1605, 1630, 1647, 1683, 2562, 2577, 2608, 2634, 2689, 2717, 2836., 2924, 2949, 2989, 3007, 3032, 3061, 3322, 3376, 3422 cm⁻¹.

2. A process for producing che polymorph (IV) of Donapezil hydrochloride as defined in claim 1, which comprises the step of humidifying the polymorph (II) as defined below:
Polymorph (II)
Peaks in the powder X-ray diffraction pattern are:
| Diffraction angles | Intensity |
|---|---|
| (2θ,°) | (I/I₀) |
| 7.40 | 8 |
| 9.88 | 100 |
| 12.36 | 13 |
| 15.54 | 40 |
| 16.10 | 38 |
| 16.22 | 38 |
| 15.48 | 35 |
| 17.30 | 17 |
| 18.04 | 20 |
| 18.44 | 17 |
| 18.84 | 19 |
| 19.34 | 19 |
| 19.84 | 47 |
| 21.16 | 24 |
| 22.40 | 19 |
| 23.18 | 33 |
| 24.02 | 22 |
| 24.92 | 25 |
| 25.72 | 27 |
| 26.40 | 18 |
| 27.22 | 14. |
Wave numbers (cm⁻¹) of infrared absorption spectra in potassium bromide are:
699, 748, 762, 845, 947, 1009, 1035, 1067, 1103, 1118, 1129, 1174, 1193, 1206, 1222, 1247, 1267, 1317, 1365, 1422, 1436, 1456, 1465, 1502, 1592, 1607, 1688, 2412, 2489, 2627, 2846, 2868, 2913, 2928, 3435 cm⁻¹.

3. A process for producing the polymorph (IV) of Donepezil hydrochloride as defined in claim 1, which comprises the steps of dissolving Donepezil in water with or without tetrahydrofuran and adding hydrochloric acid or hydrogen chloride to the solution.

4. A process for producing the polymorph (IV) of Donepezil hydrochloride as defined in claim 1, which comprises the steps of dissolving Donepezil in hydrochloric acid and adding tetrahydrofuran to the solution.

5. A process for producing the polymorph (IV) of Donepezil hydrochloride as defined in claim 1, which comprises the steps of dissolving Donepezil in toluene and adding hydrochloric acid to the solution.

6. A process for producing the polymorph (IV) of Donepezil hydrochloride as defined in claim 1, which comprises the steps of dissolving Donepazil in n-hexane and adding hydrochloric acid to the solution.

7. A process for producing the polymorph (IV) of Donepezil hydrochlorids as defined in claim 1, which comprises the step of crystallizing Donepezil in a mixture of methanol and hydrochloric acid.

8. A process for producing the polymorph (IV) of Donepezil hydrochloride as defined in claim 1, which comprises the step of crystallizing Donepezil hydrochloride from water.

9. A process for producing the polymorph (IV) of Donepezil hydrochloride as defined in claim 1, which comprises the step of humidifying the amorphous form of Donepezil hydrochloride.

10. A process for producing the polymorph (IV) of Donepezil hydrochloride as defined in claim 1, which comprises the step of humidifying the polymorph (II) of Donepezil hydrochloride.

11. Use of a pharmacologically effective amount of the Donepezil hydrochloride in the form of a polymorph as defined in claim 1 for the preparation of a medicament for the treatment of a disease accompanied by acetylcholinasterase activity.

12. Use as claimed in claim 11 in which the disease is senile dementia.

13. Use as claimed in claim 11 in which the disease is senile dementia of the Alzheimer type.

14. A therapeutical composition which comprises a pharmacologically effective amount of Donepezil hydrochloride in the form of polymorph as defined in claim 1 and a pharmacologically acceptable carrier.

## Patentansprüche

1. Donepezilhydrochlorid, 1-Benzyl-4-[(5,6-dimethoxy-1-indanon)-2-yl]methylpiperidin-hydrochlorid, in Form von Polymorph (IV), wobei der Polymorph durch Peaks bei den nachstehend gezeigten Beugungswinkeln mit der nachstehend gezeigten Intensität, ausgedrückt als I/I₀, in einem Pulverröntgenbeugungsmuster und die nachstehend gezeigten Absorptionspeaks in Infrarotabsorptionsspektren in Kaliumbromid, ausgedrückt als reziproke Zentimeter, spezifiziert ist:
Polymorph (IV)
Peaks im Pulverröntgenbeugungsmuster sind:
| Beugungswinkel | Intensität |
|---|---|
| (2θ, °) | (I/I₀) |
| 6,24 | 15 |
| 9,66 | 12 |
| 11,04 | 22 |
| 12,12 | 24 |
| 12,54 | 67 |
| 12,76 | 61 |
| 13,98 | 27 |
| 14,42 | 15 |
| 14,88 | 11 |
| 16,34 | 12 |
| 17,46 | 100 |
| 18,12 | 25 |
| 18,60 | 32 |
| 19,06 | 15 |
| 19,98 | 74 |
| 20,42 | 41 |
| 20,62 | 34 |
| 21,30 | 48 |
| 21,80 | 63 |
| 22,58 | 78 |
| 23,04 | 46 |
| 24,00 | 32 |
| 24,54 | 49 |
| 25,14 | 90 |
| 25,36 | 99 |
| 26,06 | 34 |
| 28,10 | 41 |
| 28,56 | 39 |
| 29,30 | 31 |
| 29,44 | 28 |
Wellenzahlen (cm⁻¹) von Infrarotabsorptionsspektren in Kaliumbromid sind:
401, 431, 459, 467, 490, 506, 518, 561, 586, 606, 631, 651, 709, 758, 766, 857, 944, 1009, 1041, 1106, 1119, 1132, 1213, 1225, 1265, 1304, 1318, 1429, 1458, 1470, 1500, 1589, 1605, 1630, 1647, 1683, 2562, 2577, 2608, 2634, 2689, 2717, 2836, 2924, 2949, 2989, 3007, 3032, 3061, 3322, 3376, 3422 cm⁻¹.

2. Verfahren zur Herstellung des Polymorphs (IV) von Donepezilhydrochlorid, wie in Anspruch 1 definiert, das den Schritt des Anfeuchtens des Polymorphs (II), wie im folgenden definiert, umfasst:
Polymorph (II)
Peaks im Pulverröntgenbeugungsmuster sind:
| Beugungswinkel | Intensität |
|---|---|
| (2θ, °) | (I/I₀) |
| 7,40 | 8 |
| 9,88 | 100 |
| 12,36 | 13 |
| 15,54 | 40 |
| 16,10 | 38 |
| 16,22 | 38 |
| 16,48 | 35 |
| 17,30 | 17 |
| 18,04 | 20 |
| 18,44 | 17 |
| 18,84 | 19 |
| 19,34 | 19 |
| 19,84 | 47 |
| 21,16 | 24 |
| 22,40 | 19 |
| 23,18 | 33 |
| 24,02 | 22 |
| 24,92 | 25 |
| 25,72 | 27 |
| 26,40 | 18 |
| 27,22 | 14 |
Wellenzahlen (cm⁻¹) von Infrarotabsorptionsspektren in Kaliumbromid sind:
699, 748, 762, 845, 947, 1009, 1035, 1067, 1103, 1118, 1129, 1174, 1193, 1206, 1222, 1247, 1267, 1317, 1365, 1422, 1436, 1456, 1465, 1502, 1592, 1607, 1688, 2412, 2489, 2627, 2846, 2868, 2913, 2928, 3435 cm⁻¹.

3. Verfahren zur Herstellung des Polymorphs (IV) von Donepezilhydrochlorid, wie in Anspruch 1 definiert, das die Schritte Auflösen von Donepezil in Wasser mit oder ohne Tetrahydrofuran und Zugeben von Salzsäure oder Chlorwasserstoff zu der Lösung umfasst.

4. Verfahren zur Herstellung des Polymorphs (IV) von Donepezilhydrochlorid, wie in Anspruch 1 definiert, das die Schritte Auflösen von Donepezil in Salzsäure und Zugeben von Tetrahydrofuran zu der Lösung umfasst.

5. Verfahren zur Herstellung des Polymorphs (IV) von Donepezilhydrochlorid, wie in Anspruch 1 definiert, das die Schritte Auflösen von Donepezil in Toluol und Zugeben von Salzsäure zu der Lösung umfasst.

6. Verfahren zur Herstellung des Polymorphs (IV) von Donepezilhydrochlorid, wie in Anspruch 1 definiert, das die Schritte Auflösen von Donepezil in n-Hexan und Zugeben von Salzsäure zu der Lösung umfasst.

7. Verfahren zur Herstellung des Polymorphs (IV) von Donepezilhydrochlorid, wie in Anspruch 1 definiert, das den Schritt des Kristallisierens von Donepezil in einer Mischung aus Methanol und Salzsäure umfasst.

8. Verfahren zur Herstellung des Polymorphs (IV) von Donepezilhydrochlorid, wie in Anspruch 1 definiert, das den Schritt des Kristallisierens von Donepezilhydrochlorid aus Wasser umfasst.

9. Verfahren zur Herstellung des Polymorphs (IV) von Donepezilhydrochlorid, wie in Anspruch 1 definiert, das den Schritt des Anfeuchtens der amorphen Form von Donepezilhydrochlorid umfasst.

10. Verfahren zur Herstellung des Polymorphs (IV) von Donepezilhydrochlorid, wie in Anspruch 1 definiert, das den Schritt des Anfeuchtens des Polymorphs (II) von Donepezilhydrochlorid umfasst.

11. Verwendung einer pharmakologisch wirksamen Menge von Donepezilhydrochlorid in Form eines Polymorphs, wie in Anspruch 1 definiert, für die Herstellung eines Medikaments für die Behandlung einer Erkrankung, die einhergeht mit Acetylcholinesteraseaktivität.

12. Verwendung gemäß Anspruch 11, in welcher die Erkrankung senile Demenz ist.

13. Verwendung gemäß Anspruch 11, in welcher die Erkrankung senile Demenz vom Alzheimer-Typ ist.

14. Eine therapeutische Zusammensetzung, die eine pharmakologisch wirksame Menge von Donepezilhydrochlorid in Form des in Anspruch 1 definierten Polymorphs und einen pharmakologisch akzeptablen Träger umfasst.

## Revendications

1. Chlorhydrate de Donépézile, chlorhydrate de 1-benzyl-4-[(5,6-diméthoxy-1-indanon)-2-yl]méthyl-pipéridine, sous forme d'un polymorphe (IV), le polymorphe étant spécifié par des pics aux degrés de diffraction indiqués ci-dessous, avec l'intensité indiquée ci-dessous exprimée par I/I₀ dans le diagramme de diffraction des rayons X de la poudre et des pics d'absorption indiqués ci-dessous dans les spectres d'absorption infrarouge dans le bromure de potassium exprimés en inverse de centimètres :
Polymorphes (IV)
Les pics dans le diagramme de diffraction des rayons X de la poudre sont :
| Angles de diffraction (2θ, °) | Intensité (I/I₀) |
|---|---|
| 6,24 | 15 |
| 9,66 | 12 |
| 11,04 | 22 |
| 12,12 | 24 |
| 12,54 | 67 |
| 12,76 | 61 |
| 13,98 | 27 |
| 14,42 | 15 |
| 14,88 | 11 |
| 16,34 | 12 |
| 17,46 | 100 |
| 18,12 | 25 |
| 18,60 | 32 |
| 19,06 | 15 |
| 19,98 | 74 |
| 20,42 | 41 |
| 20,62 | 34 |
| 21,30 | 48 |
| 21,80 | 63 |
| 22,58 | 78 |
| 23,04 | 46 |
| 24,00 | 32 |
| 24,54 | 49 |
| 25,14 | 90 |
| 25,36 | 99 |
| 26,06 | 34 |
| 28,10 | 41 |
| 28,58 | 39 |
| 29,30 | 31 |
| 29,44 | 28 |
Les nombres d'ondes (cm⁻¹) du spectre d'absorption infrarouge dans le bromure de potassium sont :
401, 431, 459, 467, 490, 506, 518, 561, 586, 606, 631, 651, 709, 758, 766, 857, 944, 1009, 1041, 1106, 1119, 1132, 1213, 1225, 1265, 1304, 1318, 1429, 1458, 1470, 1500, 1589, 1605, 1630, 1647, 1683, 2562, 2577, 2608, 2634, 2689, 2717, 2836, 2924, 2949, 2989, 3007, 3032, 3061, 3322, 3376, 3422 cm⁻¹.

2. Procédé de fabrication du polymorphe (IV) du chlorhydrate de Donépézile tel que défini dans la revendication 1, qui comprend l'étape consistant à humidifier le polymorphe (II) tel que défini ci-dessous :
Polymorphe (II)
Les pics dans le diagramme de diffraction des rayons X de la poudre sont :
| Diffraction angles (2θ, °) | Intensité (I/I₀) |
|---|---|
| 7,40 | 8 |
| 9,88 | 100 |
| 12,36 | 13 |
| 15,54 | 40 |
| 16,10 | 38 |
| 16,22 | 38 |
| 16,48 | 35 |
| 17,30 | 17 |
| 18,04 | 20 |
| 18,44 | 17 |
| 18,84 | 19 |
| 19,34 | 19 |
| 19,84 | 47 |
| 21,16 | 24 |
| 22,40 | 19 |
| 23,18 | 33 |
| 24,02 | 22 |
| 24,92 | 25 |
| 25,72 | 27 |
| 26,40 | 18 |
| 27,22 | 14 |
Les nombres d'ondes (cm⁻¹) du spectre d'absorption infrarouge dans le bromure de potassium sont :
699, 748, 762, 845, 947, 1009, 1035, 1067, 1103, 1118, 1129, 1174, 1193, 1206, 1222, 1247, 1267, 1317, 1365, 1422, 1436, 1456, 1465, 1502, 1592, 1607, 1688, 2412, 2489, 2627, 2846, 2868, 2913, 2928, 3435 cm⁻¹.

3. Procédé de fabrication du polymorphe (IV) du chlorhydrate de Donépézile tel que défini dans la revendication 1, qui comprend les étapes consistant à dissoudre le Donépézile dans de l'eau en présence ou en l'absence de tétrahydrofurane et à ajouter de l'acide chlorhydrique ou du chlorure d'hydrogène à la solution.

4. Procédé de fabrication du polymorphe (IV) du chlorhydrate de Donépézile tel que défini dans la revendication 1, qui comprend les étapes consistant à dissoudre du Donépézile dans de l'acide chlorhydrique et à ajouter du tétrahydrofurane à la solution.

5. Procédé de fabrication du polymorphe (IV) du chlorhydrate de Donépézile tel que défini dans la revendication 1, qui comprend les étapes consistant à dissoudre du Donépézile dans du toluène et à ajouter de l'acide chlorhydrique à la solution.

6. Procédé de fabrication du polymorphe (IV) du chlorhydrate de Donépézile tel que défini dans la revendication 1, qui comprend les étapes consistant à dissoudre du Donépézile dans du n-hexane et à ajouter de l'acide chlorhydrique à la solution.

7. Procédé de fabrication du polymorphe (IV) du chlorhydrate de Donépézile tel que défini dans la revendication 1, qui comprend l'étape de cristallisation du Donépézile dans un mélange de méthanol et d'acide chlorhydrique.

8. Procédé de fabrication du polymorphe (IV) du chlorhydrate de Donépézile selon la revendication 1, qui comprend l'étape de cristallisation du chlorhydrate de Donépézile à partir de l'eau.

9. Procédé de fabrication du polymorphe (IV) du chlorhydrate de Donépézile selon la revendication 1, qui comprend l'étape d'humidification de la forme amorphe du chlorhydrate de Donépézile.

10. Procédé de fabrication du polymorphe (IV) du chlorhydrate de Donépézile selon la revendication 1, qui comprend l'étape d'humidification du polymorphe (II) du chlorhydrate de Donezepil.

11. Utilisation d'une quantité pharmacologiquement efficace de chlorhydrate de Donépézile sous la forme du polymorphe tel que défini dans la revendication 1, pour la préparation d'un médicament pour le traitement d'une maladie accompagnée d'une activité acétylcholinestérase.

12. Utilisation selon la revendication 11, dans laquelle la maladie est une démence sénile.

13. Utilisation selon la revendication 11, dans laquelle la maladie est une démence sénile du type Alzheimer.

14. Composition thérapeutique qui comprend une quantité pharmacologiquement efficace de chlorhydrate de Donépézile sous la forme du polymorphe selon la revendication 1 et un support pharmacologiquement acceptable.
